(19) Europäisches Patentamt
European Patent Office
Office européen des brevets

(11) Veröffentlichungsnummer: **0 122 580**
**B1**

# (12) EUROPÄISCHE PATENTSCHRIFT

(45) Veröffentlichungstag der Patentschrift: **28.10.87**

(21) Anmeldenummer: **84103964.7**

(22) Anmeldetag: **09.04.84**

(51) Int. Cl.[4]: **C 07 D 401/04,** C 07 D 451/02, C 07 D 451/14, A 61 K 31/505 // (C07D401/04, 239:48, 211:26), (C07D451/14, 221:00), (C07D451/02, 221:00, 209:00)

## (54) Neue Pyrimidinderivate, deren Herstellung und pharmazeutische Präparate

(30) Priorität: **14.04.83 CH 2003/83**
**10.02.84 CH 633/84**

(43) Veröffentlichungstag der Anmeldung: **24.10.84 Patentblatt 84/43**

(45) Bekanntmachung des Hinweises auf die Patenterteilung: **28.10.87 Patentblatt 87/44**

(84) Benannte Vertragsstaaten: **AT BE CH DE FR GB IT LI LU NL SE**

(56) Entgegenhaltungen:
**DE - A - 2 609 208**
**DE - A - 3 001 328**
**FR - A - 2 493 848**
**GB - A - 762 256**
**US - A - 3 133 073**
**US - A - 4 109 092**

**CHEMICAL ABSTRACTS, vol. 88, no. 21, 22. Mai 1978, Columbus, Ohio, USA, KOMPIS, IVAN et al. "2,4-Diamino-5-(pyridylmethyl)-pyrimidine as potential chemotherapeutics", Seite 602, abstract no. 152 540f**

(73) Patentinhaber: **F. HOFFMANN-LA ROCHE & CO. Aktiengesellschaft, CH-4002 Basel (CH)**

(72) Erfinder: **Kompis, Ivan, Dr., Lettenhofstrasse 6, CH-4104 Oberwil (CH)**
Erfinder: **Locher, Rita, Dr., Riehenring 71, CH-4058 Basel (CH)**
Erfinder: **Maag, Hans, Dr., 50 Gordonhurst Avenue, Upper Montclair, N.J. 07043 (US)**

(74) Vertreter: **Lederer, Franz, Dr. et al, Vanderwerth, Lederer & Riederer Patentanwälte Lucile-Grahn-Strasse 22, D-8000 München 80 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Pyrimidinderivate der allgemeinen Formel

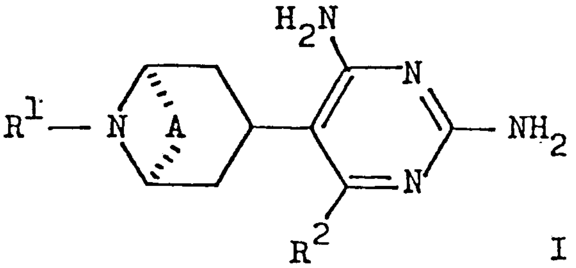

I

worin $R^1$ einen Naphthylrest oder einen Naphthyl- oder Phenylrest darstellt, der durch $C_{1-6}$-Alkyl, $C_{1-6}$-Alkylthio, $C_{1-6}$-Alkoxy, Halogen, Amino, $C_{1-6}$-Alkylamino oder di-($C_{1-6}$-Alkyl)-amino, Nitro, Trifluormethyl, $C_{1-6}$-Alkoxycarbonyl, Carboxyl, Cyano oder -CONHR$^3$, wobei $R^3$ Wasserstoff, $C_{1-6}$-Alkyl oder NHR$^3$ der über die Aminogruppe gebundene Rest von Glycin,β-Alanin, Asparaginsäure oder Glutaminsäure ist, substituiert ist,
$R^2$ Wasserstoff oder $C_{1-6}$-Alkoxy; und A einen fakultativ anwesenden Äthylenrest oder 1,3-Propylenrest darstellen, und der Diaminopyrimidinrest in Verbindungen der Formel I in denen A anwesend ist, exo-Konfiguration aufweist.

Die Erfindung betrifft weiterhin pharmazeutische Präparate, die eine Verbindung der Formel I enthalten, sowie ein Verfahren zur Herstellung der Verbindungen der Formel I und dabei verwendete Zwischenprodukte. Der Naphthylrest kann ein α-Naphthyl- oder β-Naphthylrest sein. Alkylgruppen können geradekettig oder verzweigt sein. Beispiele für solche Gruppen sind Methly, Äthyl, Propyl, Isopropyl und Butyl. Bevorzugte substituierte Phenylreste sind 4-Methoxyphenyl, 3,5-Dimethoxyphenyl und 3,4,5-Trimethoxyphenyl. Eine bevorzugte Gruppe von Verbindungen der Formel I sind die, in denen $R^1$ ein wie oben definierter substituierter Phenylrest ist. Weiterhin sind Verbindungen der Formel I bevorzugt, in denen A Äthylen ist. Diese Verbindungen können durch die allgemeine Formel

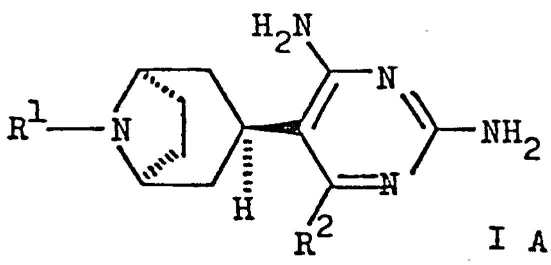

I A

dargestellt werden.

In anderer Hinsicht betrifft die Erfindung Verbindungen der allgemeinen Formel

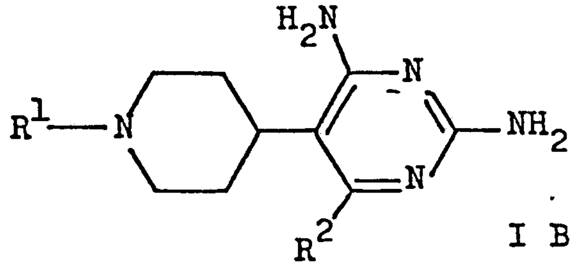

I B

Die Verbindungen der allgemeinen Formel I können erfindungsgemäss dadurch hergestellt werden, dass man

a) eine Verbindung der allgemeinen Formel

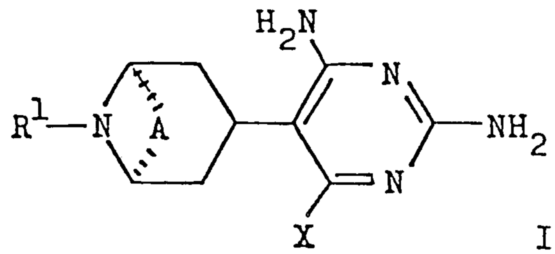

II

worin X Chlor oder Brom ist und $R^1$ und A die obige Bedeutung haben,
mit einem Reduktionsmittel behandelt, oder

b: eine Verbindung der allgemeinen Formel

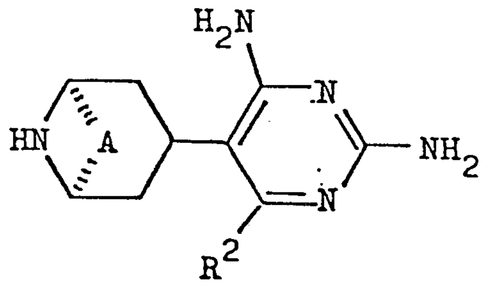

III

worin $R^2$ Wasserstoff oder $C_{1-6}$-Alkoxy ist, mit einer Verbindung der allgemeinen Formel $R^{11}$-Z umsetzt, wobei $R^{11}$ ein Naphthylrest oder ein substituierter Napthyl- oder Phenylrest ist, der durch $C_{1-6}$-Alkoxy, Halogen, Nitro, Trifluormethyl, $C_{1-6}$-Alkoxycarbonyl, oder Cyano substituiert ist oder

c) eine Verbindung der allgemeinen Formel II mit einem Alkalimetall-$C_{1-6}$-alkylat umsetzt, und gewünschtenfalls im Reaktionsprodukt einen in $R^1$ enthaltenen Substituenten in einen anderen in $R^1$ enthaltenen Substituenten abwandelt.

Geeignete Reduktionsmittel für die Entfernung des Chlor- oder Bromsubstituenten X aus einer Verbindung der Formel II sind Wasserstoff in Gegenwart von Edelmetallkatalysatoren, wie Pd/Kohle; oder nascierender Wasserstoff z.B. Zn/Eisessig, oder amalgamiertes Zink in NaOH. Die Reduktion kann in an sich bekannter Weise vorgenommen werden. Die katalytische Hydrierung wird zweckmässig bei Zimmertemperatur in einem inerten Lösungsmittel, z.B. wässriger Essigsäure, oder Äthanol durchgeführt. Die Reduktion mit nascierendem Wasserstoff wird zweckmässig unter Erwärmen, z.B. bei Temperaturen bis zur Rückflusstemperatur des Reaktionsgemisches durchgeführt.

Die N-Substitution einer Verbindung der Formel III kann in an sich bekannter Weise erfolgen. Die Umsetzung wird zweckmässig in einem inerten Lösungsmittel, wie Acetonitril oder Dimethylsulfoxid in Gegenwart eines säurebindenen Mittels, wie Alkali- oder Erdalkalihydroxiden oder -carbonaten unter Erwärmen, z.B. bis zur Rückflusstemperatur des Reaktionsgemisches, durchgeführt. Z ist vorzugsweise Halogen, insbesondere Fluor.

Die Umsetzung einer Verbindung der Formel II mit einem Alkalimetall-$C_{1-6}$-Alkylat kann durch Erwärmen der Reaktionspartner in dem dem Alkylat entsprechenden Alkohol, z.B. auf Temperaturen bis Rückflusstemperatur, vorgenommen werden. Beispiele von Alkalimetall-$C_{1-6}$-alkylaten sind Natrium- und Kaliummethylat oder -äthylat.

In den so erhaltenen Verbindungen der Formel I können in $R^1$ enthaltene Substituenten abgewandelt werden. Beispielsweise kann eine Alkoxycarbonylgruppe zur Carboxygruppe verseift oder in eine Carboxaminogruppe umgewandelt werden; eine Carboxygruppe kann zu einer Gruppe $CONHR^2$ amidiert werden; eine Nitrogruppe kann zur Aminogruppe reduziert werden, worauf die Aminogruppe alkyliert oder diazotiert und gegen ein Halogenatom ausgetauscht werden kann. Diese Reaktionen können nach Methoden und unter Bedingungen durchgeführt werden, die für die Verseifung von Estergruppen, Amidierung von Carbonsäure- oder Carbonsäureestergruppen, Reduktion von Nitrogruppen zu Aminogruppen Alkylierung der letzteren sowie Austausch von aromatischen Aminogruppen gegen andere Gruppen (Sandmeyer-Reaktion) generell bekannt sind. Verbindungen der Formel I, in denen $R^1$ Carboxyl oder $-CONHR^2$ darstellen, werden zweckmässig dadurch hergestellt, dass man eine erhaltene Verbindung der Formel I, in der $R^1$ $C_{1-6}$-Alkoxycarbonyl ist, verseift und die so erhaltene Carbonsäure gegebenenfalls mit einem $C_{1-6}$-Alkylamin oder einer Aminosäure $R^3NH_2$ in Gegenwart von Kondensationsmitteln, wie Dicyclohexylcarbodiimid oder Carbonyldiimidazol umsetzt.

Die Verbindungen der Formel II sind neu und ebenfalls Gegenstand der Erfindung. Sie können wie in den nachstehenden Formelschemata angedeutet, und in den Beispielen detailliert beschrieben, hergestellt werden.

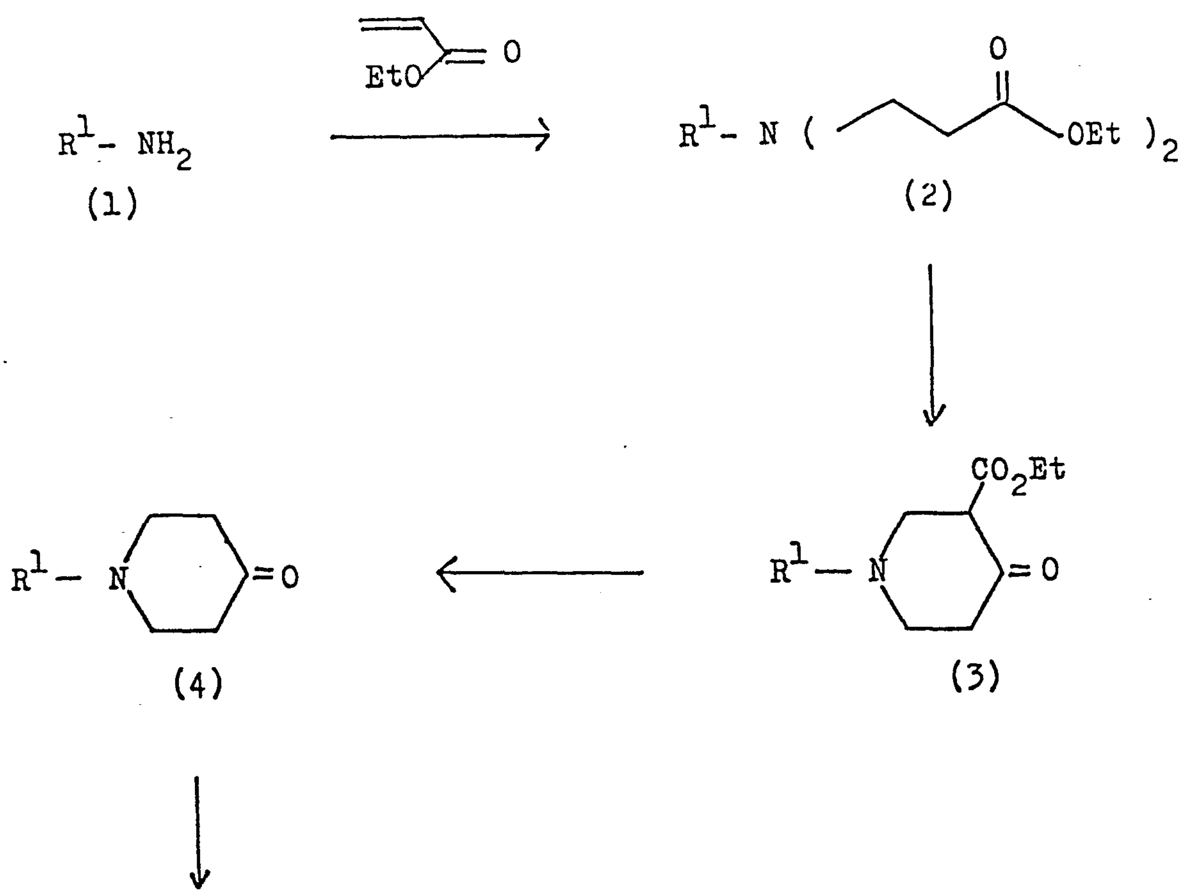

(1) (2) (3) (4)

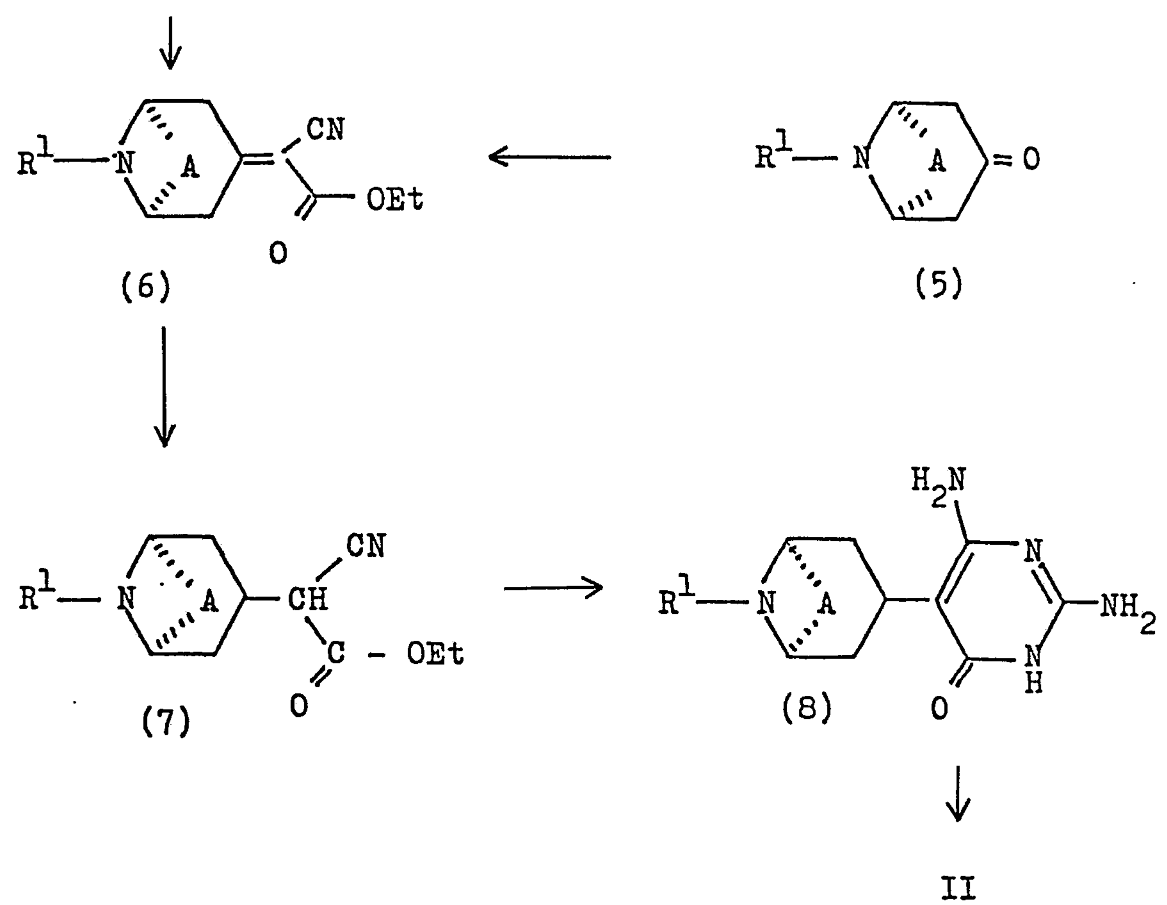

(6) (5) (7) (8) II

Durch Umsetzung eines entsprechend substituierten Anilins oder eines Naphthylamins (1) mit Acrylsäureäthylester erhält man das tertiäre Amin (2) und daraus durch Dieckmann-Kondensation Hydrolyse und Decarboxylierung des β-Ketoesters (3) das Piperidon (4). Knoevenagel-Kondensation mit Cyanessigsäureäthylester von (4) bzw. (5) (Tetrahedron 28, 155–165 [1972]) und Bull. Chem. Soc. Japan 44, 1708–9 [1971]) liefert (6), das durch Reduktion der konjugierten Doppelbindung in (7) übergeführt wird. Die Reduktion von Verbindungen (6) in denen A anwesend ist, wird zweckmässig mit $LiAlH_4$ oder $Li/NH_3$ durchgeführt, wobei man (7) als Isomerengemisch erhält, in dem der Cyanessigsäureester-Rest endo- oder exo-Konfiguration aufweist. Das für die Herstellung der bevorzugten Verbindungen IA benötigte exo-Isomer lässt sich in an sich bekannter Weise, z.B. durch Chromatographie, abtrennen. Kondensation von (7) mit Guanidin liefert das Pyrimidon (8), aus dem durch Halogenierung II erhalten werden kann. Die Umsetzung (7) → (8) → II kann in Analogie zu bekannten Verfahrensweisen, die z.B. in der deutschen Offenlegungsschrift 2 003 578 beschrieben sind, durchgeführt werden.

Die Verbindungen der Formel I sind pharmakologisch wirksam. Insbesondere sind sie antibakteriell und gegen Malariaerreger und Coccidiose wirksam. Ferner wurde eine Hemmwirkung auf das Wachstum der Tumoren S180 und L1210 festgestellt.

Die Verbindungen der Formel I hemmen die Dihydrofolatreduktase und potenzieren die antibakterielle Wirkung von Sulfonamiden. Sie können daher auch in Kombination mit antibakteriell wirksamen Sulfonamiden als Mittel zur Bekämpfung bakterieller Infektionen Verwendung finden.

Es wurde gefunden, dass das exo-2,4-Diamino-5-[8-(3,5-dimethoxyphenyl)-8-azabicyclo-[3.2.1]oct-3-yl]pyrimidin (A) bei Dihydrofolatreduktase aus E. coli in einer Hemmkonzentration $IC_{50}$ von 3,3 nMol wirksam ist. Bei in vitro Versuchen wurde für die Verbindung A gegenüber Bacteroidesfragilis-Stämmen eine minimale Hemmkonzentration von 0,1–1,6 μg/ml ermittelt. Gegenüber dem Malaria-Erreger P. falciparum zeigte die Verbindung A in vitro eine $ID_{50}$ von 1,4 μg/l (Stamm T9) und 0,9 μg/l (Stamm 13). Im Vergleich hierzu wurden für das bekannte Pyrimethamin Werte von 79 bzw. 45 μg/l ermittelt.

Beispiele von Sulfonamiden, die durch die Verbindungen der Formel I potenziert werden, sind solche der Pyrimidin-, Isoxazol-, Oxazol- und Pyrazinreihe wie Sulfadiazin, Sulfadimethoxin, Sulfadoxin, Sulfamerazin, Sulfameter, Sulfamethazin, 6-Methoxy-4-sulfanilamidopyrimidin, Sulfamethoxazol, Sulfisoxazol, 3-Sulfanilamido-4,5-dimethyl-isoxazol, Sulfamoxol und Sulfalen.

Die Verbindungen der Formel I können als pharmazeutische Präparate in Mischung mit einem für die orale, rektale oder parenterale Applikation geeigneten organischen oder anorganischen inerten Trägermaterial, z.B. Wasser, Gelatine, Gummi arabicum, Milchzucker, Stärke, Magnesiumstearat, Talk, pflanzlichen Ölen, Polyalkylenglykolen, Vaseline, usw. verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der dem Fachmann geläufigen Weise erfolgen.

Die nachstehenden Beispiele erläutern die Erfindung weiter.

Beispiel 1

180 mg exo-4-Chlor-2,6-diamino-5-[8-(4-methoxyphenyl)-8-azabicyclo[3.2.1]oct-3-yl]pyrimidin din wurden in 20 ml eines Gemisches von Essigsäure und Wasser (1:1) gelöst und in Gegenwart von 100 mg 10% Palladium/Kohle-Katalysator hydriert, bis kein Wasserstoff mehr aufgenommen wurde bzw. die Reaktion gemäss Dünnschichtchromatographie beendet war. Der Katalysator wurde durch Filtrieren über ein Filterhilfsmittel entfernt und das Filtrat zur Trockne eingedampft. Der Rückstand wurde in Wasser suspendiert und die Suspension durch Zusatz von wässrigem Ammoniumhydroxid auf pH 9 eingestellt. Filtration lieferte ein Rohprodukt, das mit Chloroform/Methanol (9:1) auf Silicagel chromatographiert wurde. Anschliessende Kristallisation aus Methanol lieferte exo-2,4-Diamino-5-[8-(4-methoxyphenyl)-8-azabicyclo[3.2.1]oct-3-yl]pyrimidin vom Schmelzpunkt 242°.

Das Ausgangsmaterial wurde wie folgt hergestellt:

A. 3,0 g 8-(4-Methoxyphenyl)-8-azabicyclo-[3.2.1]octan-3-on wurden in 6 ml Dimethylformamid gelöst und mit 1,8 ml Cyanessigsäureäthylester, 0,2 ml Piperidin und 58 mg β-Alanin versetzt. Das Gemisch wurde 28 Stunden unter Rühren auf 50° erwärmt. Nach Kühlen auf Raumtemperatur wurde das Gemisch mit Äther verdünnt, viermal mit Wasser gewaschen, über Natriumsulfat getrocknet und unter vermindertem Druck konzentriert. Chromatographie des Rückstandes an 250 g Silicagel mit Hexan/Äthylacetat (3:2) lieferte Äthyl Cyano-[8-(4-methoxyphenyl)-8-azabicyclo[3.2.1]octanyliden]acetat als weissen Feststoff, Schmelzpunkt 121–122° (aus Isopropyläther).

B. 1,82 g der so erhaltenen Verbindung wurden in 22 ml Tetrahydrofuran unter Argon gelöst. Das Gemisch wurde auf -10° gekühlt, mit 110 mg Lithiumaluminiumhydrid versetzt, 10 Minuten bei -10°C gerührt und danach mit gesättigter wässriger Ammoniumchloridlösung und Wasser versetzt. Die dicke Suspension wurde durch Filterpapier filtriert und der Rückstand gründlich mit Äthylacetat gewaschen. Die organische Phase des Filtrats wurde zweimal mit Wasser gewaschen, über Natriumsulfat getrocknet und konzentriert. Der Rückstand wurde an 200 g Silicagel (230–400 mesh) mit Hexan/Äthylacetat (3:1) chromatographiert. Man erhielt 630 mg Äthyl rac-(exo)-α-Cyano-[8-(4-methoxyphenyl)-8-azabicyclo[3.2.1 octan]octan]-3-acetat als Öl neben dem endo-Isomeren.

C. Durch Auflösen von 10 mMol Natrium in 12 ml absolutem Äthanol unter Argon wurde eine äthanolische Natriumäthoxidlösung frisch hergestellt. Die Lösung wurde mit 10 mMol Guanidinhydrochlorid versetzt und 20 Minuten bei Raumtemperatur gerührt. Danach wurden 5 mMol des vorstehend erhaltenen α-Cyanoacetats (exo-Isomer) zugesetzt. Das Gemisch wurde 4 Stunden zum Rückfluss erhitzt, auf Raumtemperatur gekühlt und über Nacht im Kühlschrank belassen. Die Feststoffe wurden abfiltriert, gut mit Wasser gewaschen und getrocknet. Man erhielt exo-2,6-Diamino-5- [8-(4-methoxyphenyl)-8-azabicyclo-[3.2.1]oct-3-yl]-4-(3H)-pyrimidinon, Schmelzpunkt über 260°C (aus Methanol/Chloroform).

D. 10 mMol des vorstehend erhaltenen Pyrimidinons wurden in 15 ml $POCl_3$ suspendiert und mit 2,6 ml N,N-Dimethylanilin versetzt. Das Gemisch wurde 1,5 Stunden zum Rückfluss erhitzt (105°C), und auf Raumtemperatur abgekühlt. Etwa 2/3 des Phosphoroxychlorids wurden unter vermindertem Druck entfernt. Der viskose Rückstand wurde vorsichtig auf Eis gegeben und die erhaltene wässrige Suspension wurde bei Raumtemperatur 5 Tage stehengelassen. Das pH des Reaktionsgemisches wurde durch Zusatz von 25%iger wässriger Ammoniumhydroxidlösung auf etwa 10 eingestellt, das Produkt abfiltriert und mit Wasser und nachfolgend mit Äthanol gewaschen. Danach wurde das Produkt an Silicagel mit Chloroform/Methanol (9:1) chromatographiert. Aus 310 mg exo-2,6-Diamino-5-[8-(4-methoxyphenyl)-azabicyclo [3.2.1]oct-3-yl]-4-(3H)-pyrimidinon wurden 180 mg exo-4-Chlor-2,6-diamino-5-[8-(4-methoxyphenyl)-8-azabicyclo-[3.2.1]oct-3-yl]pyrimidin erhalten.

Beispiel 2

In Analogie zu Beispiel 1 wurden aus 1,6 g exo-4-Chlor-2,6-diamino-5-[8-(3,5-dimethoxyphenyl)-8-azabicyclo-[3.2.1]oct-3-yl]pyrimidin 980 mg exo-2,4-Diamino-5- [8-(3,5-dimethoxyphenyl)-8-azabicyclo[3.2.1]oct-3-yl]pyrimidin, Schmelzpunkt 261–262°C (aus Methanol) erhalten.

Das Ausgangsmaterial wurde in Analogie zu den in Beispiel 1 Absatz A–D beschriebenen Verfahren ausgehend von 8-(3,5-Dimethoxyphenyl)-8-azabicyclo[3.2.1]octan-3-on über

Äthyl Cyano-[8-(3,5-dimethoxyphenyl)-8-azabicyclo[3.2.1]octanyliden]acetat, Schmelzpunkt 116°C (aus Isopropyläther),

Äthyl rac-(exo)-α-cyano-[8-(3,5-dimethoxyphenyl)-8-azabicyclo[3.2.1]octan-3]acetat, Smp. 92–93°C (aus Äthanol) und

exo-2,6-Diamino-5- [8-(3,5-dimethoxyphenyl)-8-azabicyclo [3.2.1] oct-3-yl]-4-(3H)-pyrimidinon, Schmelzpunkt 193–194°C (aus Äthanol/Wasser) hergestellt.

Das Äthyl Cyano-[8-(3,5-dimethoxyphenyl)-8-azabicyclo[3.2.1]octanyliden]acetat kann wie folgt reduziert werden:

Bei -78°C wurden 0,7 g Lithium in 150 ml trokkenem, destilliertem Ammoniak gelöst und unter Stickstoff tropfenweise während 20 Minuten mit einer Lösung von 8,9 g Äthyl Cyano-[8-(3,5-dimethoxyphenyl)-8-azabicyclo[3.2.1]octanyliden]acetat und 2,35 g Phenol in 50 ml trockenem Tetrahydrofuran versetzt. Man liess während 30 Minuten rühren, worauf 5 g Ammoniumchlorid zugegeben wurden. Das Ammoniak wurde abdestilliert, der Rückstand in Essigester aufgenommen, filtriert und das Filtrat eingedampft. Chromatographie des Rohproduktes an Silicagel mit Hexan/Äthylacetat (4:1) ergab neben wenig endo-Isomeren 7,8 g Äthyl rac-(exo)-α-cyano-[8-(3,5-dimethoxyphenyl)-8-azabicyclo[3.2.1]octan-3]-acetat vom Schmelzpunkt 92–93°C (aus Äthanol).

Beispiel 3

21,5 g 2,4-Diamino-6-chlor-5-[1,(4-methoxyphenyl)-4-piperidinyl]pyrimidin wurden in 350 ml eines Gemisches von Essigsäure und Wasser (1:1) gelöst und in Gegenwart von 7 g 10% Palladium/Kohle-Katalysator hydriert, bis die Wasserstoffaufnahme aufhörte bzw. die Reaktion gemäss Dünnschichtchromatographie vollständig war. Der Katalysator wurde über ein Filterhilfsmittel abfiltriert und das Filtrat zur Trockne eingedampft. Der Rückstand wurde in Wasser suspendiert und das pH durch Zusatz von wässrigem Ammoniumhydroxid auf 9 eingestellt. Filtration lieferte ein Rohprodukt, das auf Silicagel mit Chloroform/Methanol (19:1) chromatographiert wurde. Man erhielt 8,7 g 2,4-Diamino-5-[1-(4-Methoxyphenyl)-4-piperidinyl]pyrimidin vom Schmelzpunkt 224–225°C.

Das Ausgangsmaterial wurde wie folgt hergestellt:

A. Zu einer frisch hergestellten Lösung von Natriumäthoxid in absolutem Äthanol (2,3 g Natrium in 55 ml Äthanol) wurden unter Argon 32,3 g Diäthyl 3,3'-[(4-methoxyphenyl)-imino]dipropionat in 40 ml Xylol gegeben. Das Gemisch wurde langsam auf etwa 110°C erwärmt, wobei das Äthanol abdestilliert wurde. Danach wurde noch 2 Stunden erwärmt. Das Gemisch wurde auf Raumtemperatur abgekühlt und auf Eis gegossen. Nach Neutralisieren mit konzentrierter Salzsäure wurde mit Äther extrahiert. Der Extrakt wurde dreimal mit Wasser gewaschen, über Natriumsulfat getrocknet und eingedampft. Das Rohprodukt wurde an Silicagel mit Hexan/Äthylacetat (9:1) chromatographiert und lieferte Äthyl 1-(p-methoxyphenyl)-4-oxo-3-piperidincarboxylat als gelbes Öl.

B. 57,9 g des so erhaltenen Carboxylats wurden in 225 ml 6N Salzsäure 1 Stunde zum Rückfluss erhitzt. Nach Kühlen etwa auf 2°C wurde das Gemisch durch Zusatz von 110 ml 50%iger wässriger Natronlauge neutralisiert und mit Äther extrahiert. Die organische Phase wurde dreimal mit Wasser gewaschen, über Natriumsulfat getrocknet und konzentriert. Der Rückstand wurde aus Äthanol umkristallisiert. Man erhielt 1-(p-Methoxyphenyl)-4-piperidon, Schmelzpunkt 69°C (aus Äthanol).

C. 20,5 g 1-(p-Methoxyphenyl)-4-piperidon wurden in 200 ml Benzol gelöst und mit 1,2 g Essigsäure, 0,85 ml Piperidin und 14,5 g Cyanessigsäureäthylester versetzt. Das Gemisch wurde 3 Stunden zum Rückfluss erhitzt, wobei das Reaktionswasser mittels einer Dean-Stark-Falle entfernt wurde. Nach Abkühlen auf Raumtemperatur wurde das Gemisch mit Äther verdünnt und dreimal mit 2N Natriumbicarbonatlösung und einmal mit Wasser gewaschen. Der organische Extrakt wurde über Natriumsulfat getrocknet und konzentriert. Der ölige Rückstand wurde mit Äthanol verrieben und über Nacht im Kühlschrank gelassen. Die Feststoffe wurden abfiltriert und mit Äthanol und Isopropyläther gewaschen, wobei man 24,1 g Rohprodukt erhielt. Umkristallisation aus Äthanol lieferte Äthyl Cyano-[1-(p- methoxyphenyl)-4-piperidinyliden]acetat, Schmelzpunkt 87°C.

D. 22,5 g der so erhaltenen Verbindung wurden in 500 ml Äthanol und 200 ml Methanol gelöst mit 5 g 10% Palladium-Kohle-Katalysator (50%ig in Wasser) unter kräftigem Rühren bei Raumtemperatur bis zum Ende der Wasserstoffaufnahme hydriert. Nach Filtration über ein Filterhilfsmittel wurde das Filtrat unter vermindertem Druck eingedampft und der Rückstand aus wenig Isopropyläther kristallisiert. Man erhielt Äthyl α-Cyano-[1-(4-methoxyphenyl)-4-piperidin]-acetat, Schmelzpunkt 65°C.

E. Die so erhaltene Verbindung wurde gemäss Beispiel 1, Absatz C) mit Guanidin kondensiert. Man erhielt 2,6-Diamino-5-[1-(4-methoxyphenyl)-4-piperidinyl]-4-(3H)-pyrimidinon, Schmelzpunkt 308°C (aus Dimethylformamid/Wasser).

F. In Analogie zu Beispiel 3, Absatz C) wurde aus dem so erhaltenen Pyrimidinon das 2,4-Diamino-6-chlor-5- [1-(4-methoxyphenyl)-4-piperidinyl]pyrimidin, Schmelzpunkt 254°C (Zersetzung, aus Äthanol) erhalten.

Beispiel 4

In Analogie zu Beispiel 3 wurden aus 5,7 g 2,4-Diamino-6-chloro-5-[1-(3,5-dimethoxyphenyl)-4-piperidinyl]pyrimidin 4,8 g rohes 2,4-Diamino-5-[1-(3,5-dimethoxyphenyl)-4-piperidinyl]pyrimidin erhalten, woraus nach Chromatographie und Umkristallisation aus Methanol 3,2 g reines Produkt vom Schmelzpunkt 217°C erhalten wurde.

Das Ausgangsmaterial wurde wie folgt hergestellt:

A. Ein Gemisch von 50,4 g 3,5-Dimethoxyanilin, 6,0 g Kupfer-(I)-chlorid, 46,0 g Essigsäure und 100 g Äthylacrylat wurde 19 Stunden unter Rühren zum Rückfluss erhitzt. Nach Abkühlen auf Raumtemperatur wurde das Gemisch mit etwa 30 ml Methylenchlorid verdünnt und nacheinander dreimal mit 300 ml Wasser, dreimal mit 300 ml 10%igem wässrigem Ammoniumhydroxid und wieder dreimal mit 300 ml Wasser gewaschen.

Die organische Phase wurde über Natriumsulfat getrocknet und unter vermindertem Druck eingedampft. Der Rückstand wurde an 1,5 kg Silicagel (230–400 mesh) mit Hexan/Äthylacetat (4:1) unter mittlerem Druck chromatographiert. Man erhielt 70,9 g Diäthyl-3,3'-(3,5-dimethoxyphenyl)-imino]dipropionat als gelbes Öl, Kp. 139°C/0,09 mm.

B. Aus Diäthyl-3,3'-[(3,5-dimethoxyphenyl)-imino]-dipropionat wurden in Analogie zu Beispiel 3, Absatz A) das Äthyl 1-(3,5-dimethoxyphenyl)-4-oxo-3-piperidincarboxylat, Schmelzpunkt 60–61°C erhalten.

C. 12,52 g des vorstehend genannten Carboxylats wurden unter kräftigem Rühren 2 Stunden in 81,6 ml 1N Natronlauge auf dem Dampfbad erwärmt. Nach Kühlen auf Raumtemperatur wurde das Gemisch mit Äther extrahiert, der Extrakt zweimal mit Wasser gewaschen, über Natriumsulfat getrocknet und eingedampft. Der Rückstand wurde auf Silicagel mit Hexan/Äthylacetat (4:1) chromatographiert und lieferte 4,8 g 1-(3,5-Dimethoxyphenyl)-4-piperidon als gelbes Öl.

D. In Analogie zu Beispiel 3, Absatz C) wurden aus dem vorstehend erhaltenen Piperidon das Äthyl Cyano-[1-(3,5-dimethoxyphenyl)-4-piperidinyliden]acetat, Schmelzpunkt 115°C (aus Methanol) erhalten.

E. 19,2 g der vorstehend genannten Verbindung wurden in 400 ml Äthylacetat gelöst und mit 6 g 10% Palladium/Kohle-Katalysator (50%ig in Wasser) hydriert. Man erhielt 19,7 g Äthyl α-cyano[1-(3,5-dimethoxyphenyl)-4-piperidin]-acetat als Öl.

F. Äthyl α-Cyano-[1-(3,5-dimethoxyphenyl)-4-piperidin]-acetat wurde in Analogie zu Beispiel 1, Absatz C) mit Guanidin kondensiert. Man erhielt das 2,6-Diamino-5-[1-(3,5-dimethoxyphenyl)-4-piperidinyl]-4-(3H)-pyrimidinon, Schmelzpunkt über 250°C (aus Dimethylformamid/Wasser).

G. 2,6-Diamino-5-[1-(3,5-dimethoxyphenyl)-4-piperidinyl]-4-(3H)-pyrimidinon wurde in Analogie zu Beispiel 3, Absatz D) in 2,4-Diamino-6-chlor-5-[1-(3,5-dimethoxyphenyl)-4-piperidinyl]pyrimidin, Schmelzpunkt 241–242°C (aus Methanol/Chloroform) übergeführt.

Beispiel 5

In Analogie zu Beispiel 1 wurden aus 0,4 g exo-4-Chlor-2,6-diamino-5-[8-(3,4,5-trimethoxyphenyl)-8-azabicyclo[3.2.1]oct-3-yl]pyrimidin 250 mg exo-2,4-Diamino-5-[8-(3,4,5-trimethoxyphenyl)-8-azabicyclo[3.2.1]oct-3-yl]-pyrimidin, Smp. 247–248°C (aus Methanol) erhalten.

Das Ausgangsmaterial wurde in Analogie zu den in Beispiel 1, Absatz A–D beschriebenen Verfahren ausgehend von 8-(3,4,5-Trimethoxyphenyl)-8-azabicyclo[3.2.1]octan-3-on, Smp. 144–145°C (aus Methanol) über
Äthyl Cyano-[8-(3,4,5-trimethoxyphenyl)-8-azabicyclo[3.2.1]octanyliden]acetat, Smp. 107–108°C,
Äthyl rac-(exo)-α-cyan-[8-(3,4,5-trimethoxyphenyl)-8-azabicyclo[3.2.1]octan-3]acetat (gelbliches Öl) und
exo-2,6-Diamino-5-[3,4,5-trimethoxyphenyl)-8-azabicyclo[3.2.1]oct-3-yl]-4-[3H]-pyrimidinon, Smp. 265°C (Zers.) (aus Methanol-Wasser) hergestellt.

Beispiel 6

In Analogie zu Beispiel 3 wurden aus 5,6 g 2,4-Diamino-6-chlor-5-[1-(3,4,5-trimethoxyphenyl)-4-piperidinyl]pyrimidin, 1,0 g 2,4-Diamino-5-[1-(3,4,5-trimethoxyphenyl)-4-piperidinyl]pyrimidin, Smp. 239–240°C (aus Methanol) erhalten.

Das Ausgangsmaterial wurde in Analogie zu den in Beispiel 3, Absatz C–F beschriebenen Verfahren aus 1-(3,4,5-Trimethoxyphenyl)-4-piperidon über
Äthyl-2-cyano-2-[1-(3,4,5-trimethoxyphenyl)-4-piperidinyliden]acetat, Smp. 111°C, gelbe Kristalle aus Isopropanol,
Äthyl-2-cyano-2-[1-(3,4,5-trimethoxyphenyl)-4-piperidinyl]acetat, farbloses Öl und 2,6-Diamino-5-[1-(3,4,5-trimethoxyphenyl)-4-piperidinyl]-4-3(H)-pyrimidinon, Smp. >250°C (Zers.) (aus Äthanol) hergestellt.

Beispiel 7

Ein Gemisch von 1 g 2,4-Diamino-5-(4-piperidyl)-pyrimidin, 0,71 g 4-Fluornitrobenzol und 0,7 g Kaliumcarbonat in 20 ml Acetonitril wurden unter Feuchtigkeitsausschluss während 72 Stunden am Rückfluss gekocht. Nach Abkühlen auf Raumtemperatur wurde abfiltriert, der Rückstand mit Wasser, Methanol und Äther gewaschen und aus Dimethylsulfoxid umkristallisiert. Dies ergab 1,36 g 2,4-Diamino-5-[1-(p-nitrophenyl)-4-piperidinyl]-pyrimidin. Schmelzpunkt über 250°C.

Herstellung des Ausgangsmaterials:

Eine Lösung von 26 g 2,4-Diamino-5-(1-benzyl-4-piperidinyl)pyrimidin in 700 ml Äthanol und 20 ml Essigsäure wurde über 5 g Pd/C 10% bei Raumtemperatur und Normaldruck bis zum Stillstand der Wasserstoffaufnahme hydriert, worauf vom Katalysator abfiltriert wurde. Das Filtrat wurde eingeengt und der kristalline Rückstand in 100 ml Wasser gelöst. Chromatographie an Dowex 1 (X10 200–400 mesh) ergab 2,4-Diamino-5-(4-piperidyl)-pyrimidin vom Schmelzpunkt 230–231°C (Zers.) (aus Wasser).

Beispiel 8

1 g 2,4-Diamino-5-[1-(p-nitrophenyl)-4-piperidinyl]-pyrimidin wurde in 50 ml Wasser und 10 ml konz. Salzsäure gelöst, mit 700 mg Eisenpulver versetzt und während 45 Minuten bei 80°C gerührt. Das Gemisch wurde über ein Filterhilfsmittel abfiltriert, das Filtrat auf 0–5°C abgekühlt, mit 210 mg Natriumnitrit in 2 ml Wasser diazotiert und die resultierende Diazoniumlösung bei 0–5°C zu einer Lösung von Kupfer(I)chlorid (hergestellt aus 250 mg Kupfer(II)sulfat-pentahydrat, 80 mg Natriumchlorid und 70 mg Natriumbisulfit) in 0,8 ml Salzsäure getropft. Danach wurde das Reaktionsgemisch während 1 Stunde bei 90°C erwärmt, mit Aktivkohle versetzt, filtriert und bis auf 50 ml eingeengt. Der Rückstand wurde mit Ammoniumhydroxid neutralisiert, abfiltriert und

das nach Abdestillieren des Wassers erhaltene Rohprodukt an Kieselgel mit $CHCl_3$/EtOH (9:1) chromatographiert. Umkristallisation aus Äthanol lieferte 2,4-Diamino-5-[1-(p-chlorphenyl)-4-piperidinyl]-pyrimidin vom Schmelzpunkt 254-255°C.

Beispiel 9

In Analogie zu Beispiel 7 wurden aus 2,0 g 2,4-Diamino-5-(4-piperidyl)-pyrimidin und 1,7 g 4-Fluorbenzoesäureäthylester, 3,1 g Äthyl p-[1-(2,4-diamino-5-pyrimidinyl)-4-piperidinyl]benzoat, Smp. über 250°C (aus Dimethylsulfoxid) erhalten.

Beispiel 10

2,1 g des in Beispiel 9 erhaltenen Benzoats wurde in einem Gemisch von 15 ml konz. Salzsäure und 20 ml Wasser gelöst und über Nacht bei 60°C gerührt. Die resultierende Suspension wurde auf Raumtemperatur abgekühlt, durch Zusatz von Ammoniak auf pH8 eingestellt und filtriert. Waschen des Rückstandes mit Wasser und Trocknen ergaben p-[1-(2,4-Diamino-5-pyrimidinyl)-4-piperidinyl]benzoesäure vom Schmelzpunkt 310-311°C (unter Zersetzung).

Beispiel 11

In Analogie zu Beispiel 7 wurden aus 500 mg 2,4-Diamino-5-(4-piperidinyl)-pyrimidin und 300 mg 4-Fluorbenzonitril in 10 ml Dimethylsulfoxid 250 mg 2,4-Diamino-5-[1-(p-cyanophenyl)-4-piperidinyl]-pyrimidin, Smp. >250°C (aus Dimethylsulfoxid) erhalten.

Beispiel 12

In Analogie zu Beispiel 1 wurden aus 4,1 g exo-4-Chlor-2,6-diamino-5-[8-(2,5-dimethoxyphenyl)-8-azabicyclo-[3.2.1]oct-3-yl]pyrimidin 2,4 g exo-2,4-Diamino-5-[8-(2,5-dimethoxyphenyl)-8-azabicyclo[3.2.1.]oct-3-yl]-pyrimidin, Smp. 270°C (aus Methanol) erhalten.

Das Ausgangsmaterial wurde in Analogie zu den in Beispiel 1, Absatz A–D beschriebenen Verfahren ausgehend von 8-(2,5 dimethoxyphenyl)-8-azabicyclo[3.2.1]octan-3-on, Smp. 66-67°C (aus Äther) über
Äthyl Cyano-[8-(2,5 dimethoxyphenyl)-8-azabicyclo-[3.2.1]oct-anyliden]acetat, (gelbliches Öl)
Äthyl rac-(exo)-α-cyan-[8-(2,5 dimethoxyphenyl)-8-azabicyclo[3.2.1]octan-3]acetat (Öl) und exo-2,6-Diamino-5-[8](2,5 dimethoxyphenyl)-8-azabicyclo[3.2.1]oct-3-yl]-4-[3H]-pyrimidinon, Smp. 164°C (aus Methanol-Wasser) hergestellt.

Beispiel 13

In Analogie zu Beispiel 1 wurden aus 0,18 g exo-4-Chlor-2,6-diamino-5[8-(1-naphthyl)-8-azabicyclo[3.2.1]oct-3-yl]pyrimidion 55 mg exo-2,4-Diamino-5-[8-(1-naphthyl)-8-azabicyclo[3.2.1]-oct-3-yl]-pyrimidin, Smp. 245-248°C (aus Methanol) erhalten.

Das Ausgangsmaterial wurde in Analogie zu den in Beispiel 1, Absatz A–D beschriebenen Verfahren ausgehend von 8-(1-naphthyl)-8-azabicyclo[3.2.1]octan-3-on, Smp. 114-115°C über
Äthyl Cyano-[8-(1-napthyl)-8-azabicyclo[3.2.1]-octan-yliden]-acetat, Smp. 111-114°C,
Äthyl rac-(exo)-α-cyan-[8-(1-naphthyl)-8-azabicyclo[3.2.1]octan-3]acetat (gelbliches Öl) und exo-2,6-Diamino-5-[8-(1-naphthyl)-8-azabicyclo [3.2.1]oct-3-yl]-4-[3H]-pyrimidinon Smp. 275°C (Zers.) (aus Methanol-Wasser) hergestellt.

Beispiel 14

In Analogie zu Beispiel 1 wurden aus 0,3 g exo-4-Chlor-2,6-diamino-5-[8-(2-naphthyl)-8-azabicyclo[3.2.1]oct-3-yl]pyrimidin 200 mg exo-2,4-Diamino-5-[8-(2-naphthyl)-8-azabicyclo[3.2.1]oct-3-yl]pyrimidin, Smp. 258-259°C (aus Methanol) erhalten.

Das Ausgangsmaterial wurde in Analogie zu den in Beispiel 1, Absatz A–D beschriebenen Verfahren ausgehend von 8-(2-naphthyl)-8-azabicyclo[3.2.1]octan-3-on, Smp. 120-121°C über
Äthyl Cyano-[8-(2-naphthyl)-8-azabicyclo[3.2.1]-octanyliden]-acetat, Smp. 122-123°C,
Äthyl rac-(exo)-α-cyan-[8-(2-naphthyl)-8-azabicyclo-[3.2.1]octan-3]acetat, Smp. 126-127°C und exo-2,6-Diamino-5-[8-(2-naphthyl)-8-azabicyclo [3.2.1]oct-3-yl]-4-[3H]-pyrimidinon, Smp. 275°C (Zers.) (aus Methanol-Wasser) hergestellt.

**Patentansprüche für die Vertragsstaaten: BE, CH, DE, FR, GB, IT, LI, LU, NL, SE**

1. Verbindungen der allgemeinen Formel

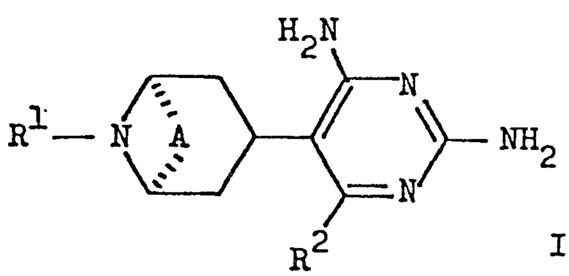

I

worin $R^1$ einen Naphthylrest oder einen Naphthyl- oder Phenylrest darstellt, der durch $C_{1-6}$-Alkyl, $C_{1-6}$-Alkylthio, $C_{1-6}$-Alkoxy, Halogen, Amino, $C_{1-6}$-Alkylamino oder di-($C_{1-6}$-Alkyl)-amino, Nitro, Trifluormethyl, $C_{1-6}$-Alkoxycarbonyl, Carboxyl, Cyano oder -CONHR³, wobei $R^3$ Wasserstoff, $C_{1-6}$-Alkyl oder $NHR^3$ der über die Aminogruppe gebundene Rest von Glycin, β-Alanin, Asparaginsäure oder Glutaminsäure ist, substituiert ist, $R^2$ Wasserstoff oder $C_{1-6}$-Alkoxy; und A einen fakultativ anwesenden Äthylenrest oder 1,3-Propylenrest darstellen, und der Diaminopyrimidinrest in Verbindungen der Formel I in denen A anwesend ist, exo-Konfiguration aufweist.

2. Verbindungen gemäss Anspruch 1, in denen $R^1$ ein wie in Anspruch 1 definierter substituierter Phenylrest ist.

3. Verbindungen gemäss Anspruch 1 der allgemeinen Formel

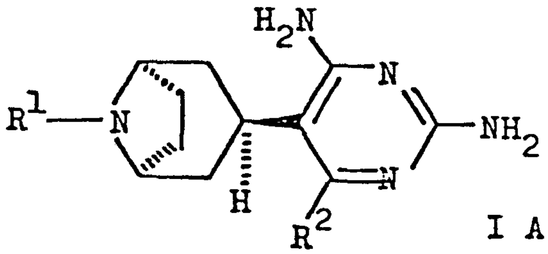

I A

worin $R^1$ die in Anspruch 2 angegebene Bedeutung hat und $R^2$ Wasserstoff oder $C_{1-6}$-Alkoxy ist.

4. Verbindungen gemäss Anspruch 1 der allgemeinen Formel

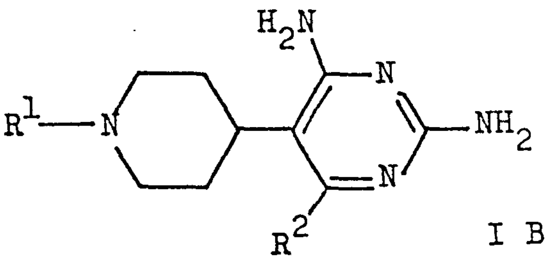

I B

worin $R^1$ die in Anspruch 2 angegebene Bedeutung hat und $R^2$ Wasserstoff oder $C_{1-6}$-Akoxy ist.

5. exo-2,4-Diamino-5-[8-(3,5-dimethoxyphenyl)-8-azabicyclo[3.2.1]oct-3-yl]pyrimidin.

6. exo-2,4-Diamino-5-[8-(4-methoxyphenyl)-8-azabicyclo[3.2.1]oct-3-yl]pyrimidin, exo-2,4-Diamino-5-[8-(3,4,5-trimethoxyphenyl)-8-azabicyclo[3.2.1]oct-3-yl]pyrimidin, exo-2,4-Diamino-5-[8-(2,5-dimethoxyphenyl)-8-azabicyclo[3.2.1]oct-3-yl]-pyrimidin, exo-2,4-Diamino-5-[8-(1-naphthyl)-8-azabicyclo[3.2.1]oct-3-yl]-pyrimidin, exo-2,4-Diamino-5-[8-(-2-napthyl)-8-azabicyclo[3.2.1]oct-3-yl]-pyrimidin.

7. 2,4-Diamino-5-[1-(4-methoxyphenyl)-4-piperidinyl]-pyrimidin, 2,4-Diamino-5-[1-(3,5-dimethoxyphenyl)-4-piperidinyl]pyrimidin, 2,4-Diamino-5-[1-(3,4,5-trimethoxyphenyl)-4-piperidinyl]pyrimidin, 2,4-Diamino-5-[1-(p-nitrophenyl)-4-piperidinyl]-pyrimidin, 2,4-Diamino-5-(4-piperidyl)-pyrimidin, 2,4-Diamino-5-[1-(p-chlorphenyl)-4-piperidinyl]-pyrimidin, Äthyl p-[1-(2,4-diamino-5-pyrimidinyl)-4-piperidinyl]-benzoat, p-[1-(2,4-Diamino-5-pyrimidinyl)-4-piperidinyl]-benzoesäure, 2,4-Diamino-5-[1-(p-cyanophenyl)-4-piperidinyl]-pyrimidin.

8. Verbindungen der Formel

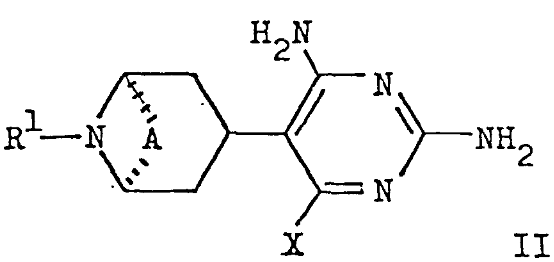

II

worin $R^1$ und A die in Anspruch 1 angegebene Bedeutung haben und X Chlor oder Brom ist.

9. Verbindungen gemäss den Ansprüchen 1–7 zur Anwendung als Heilmittel.

10. Verbindungen der allgemeinen Formel I zur Anwendung bei der Behandlung bakterieller Infektionen oder der Malaria.

11. Verfahren zur Herstellung der Verbindungen von Anspruch 1, dadurch gekennzeichnet, dass man

a) eine Verbindung der allgemeinen Formel

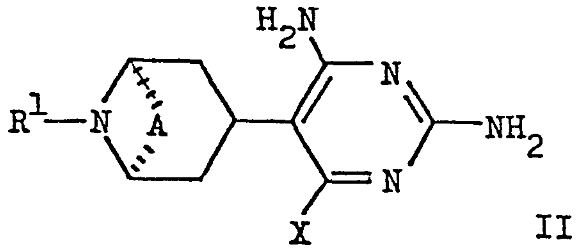

II

worin X Chlor oder Brom ist und $R^1$ und A die in Anspruch 1 angegebene Bedeutung haben, mit einem Reduktionsmittel behandelt, oder

b) eine Verbindung der allgemeinen Formel

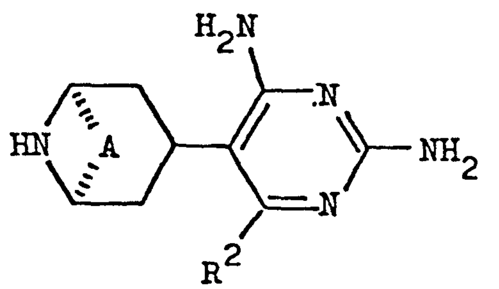

III

worin $R^2$ Wasserstoff oder $C_{1-6}$-Alkoxy ist, mit einer Verbindung der allgemeinen Formel $R^{11}$-Z umsetzt, wobei $R^{11}$ ein Naphthylrest oder ein Naphthyl- oder Phenylrest ist, der durch $C_{1-6}$-Alkoxy- Halogen, Nitro- Trifluormethyl, Cyano oder $C_{1-6}$-Alkoxycarbonyl substituiert ist und Z Halogen oder Nitro bedeutet, oder

c) eine Verbindung der allgemeinen Formel II mit einem Alkalimetall-$C_{1-6}$-alkylat umsetzt, und gewünschtenfalls im Reaktionsprodukt einen in $R^1$ enthaltenen Substituenten in einen anderen in $R^1$ enthaltenen Substituenten abwandelt.

12. Pharmazeutisches Präparat, enthaltend eine Verbindung der allgemeinen Formel I und einen Trägerstoff.

**Patentansprüche für den Vertragsstaat Österreich**

1. Verfahren zur Herstellung von Verbindungen der allgemeinen Formel

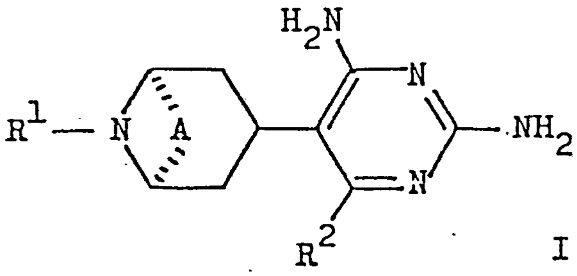

I

worin $R^1$ einen Naphthylrest oder einen Naphthyl- oder Phenylrest darstellt, der durch $C_{1-6}$-Alkyl, $C_{1-6}$-Alkylthio, $C_{1-6}$-Alkoxy, Halogen, Amino, $C_{1-6}$-Alkylamino oder di-($C_{1-6}$-Alkyl)-amino, Nitro, Trifluormethyl, $C_{1-6}$-Alkoxycarbonyl,Carboxyl, Cyano oder -$CONHR^3$, wobei $R^3$ Wasserstoff, $C_{1-6}$-Alkyl oder
$NHR^3$ der über die Aminogruppe gebundene Rest von Glycin, β-Alanin, Asparaginsäure oder Glutaminsäure ist, substituiert ist; $R^2$ Wasserstoff oder $C_{1-6}$-Alkoxy; und A einen fakultativ anwesenden Äthylenrest oder 1,3-Propylenrest darstellen, und der Diaminopyrimidinrest in Verbindungen der Formel I, in denen A anwesend ist, exo-Konfiguration aufweist, dadurch gekennzeichnet, dass man

a) eine Verbindung der allgemeinen Formel

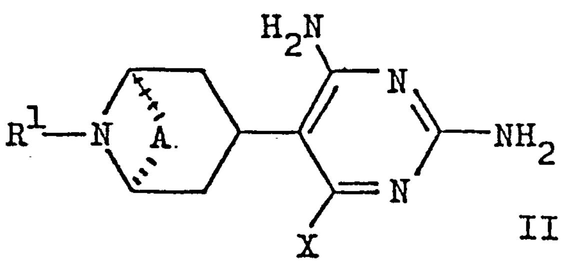

II

worin X Chlor oder Brom ist und $R^1$ und A die in Anspruch 1 angegebene Bedeutung haben, mit einem Reduktionsmittel behandelt, oder

b) eine Verbindung der allgemeinen Formel

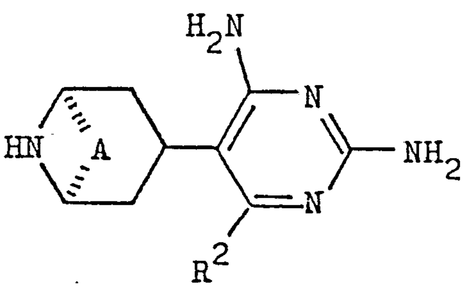

III

worin $R^2$ Wasserstoff oder $C_{1-6}$-Alkoxy ist, mit einer Verbindung der allgemeinen Formel $R^{11}$-Z umsetzt, wobei $R^{11}$ ein Naphthylrest oder ein Naphthyl- oder Phenylrest ist, der durch $C_{1-6}$-Alkoxy, Halogen, Nitro, Trifluormethyl, Cyano oder $C_{1-6}$-Alkoxycarbonyl substituiert ist und Z Halogen oder Nitro bedeutet, oder

c) eine Verbindung der allgemeinen Formel II mit einem Alkalimetall- $C_{1-6}$-alkylat umsetzt, und gewünschtenfalls im Reaktionsprodukt einen in $R^1$ enthaltenen Substituenten in einem anderen in $R^1$ enthaltenen Substituenten abwandelt.

2. Verfahren nach Anspruch 1 zur Herstellung von Verbindungen der allgemeinen Formel I in der $R^1$ ein wie in Anspruch 1 definierter substituierter Phenylrest ist.

3. Verfahren nach den Ansprüchen 1 oder 2 zur Herstellung von Verbindungen der allgemeinen Formel

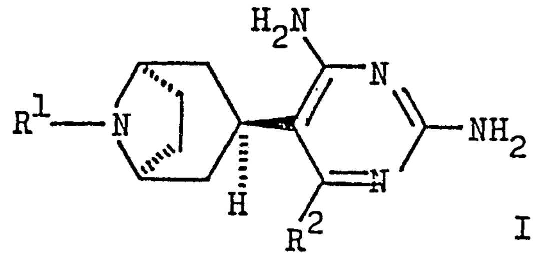

I A

worin $R^1$ die in Anspruch 2 angegebene Bedeutung hat und $R^2$ Wasserstoff oder $C_{1-6}$-Alkoxy ist.

4. Verfahren nach den Ansprüchen 1 oder 2 zur Herstellung von Verbindungen der allgemeinen Formel

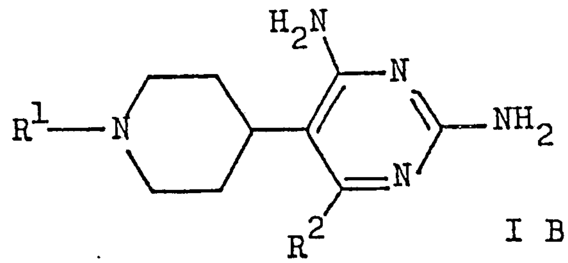

I B

worin $R^1$ die in Anspruch 2 angegebene Bedeutung hat und $R^2$ Wasserstoff oder $C_{1-6}$-Alkoxy ist.

5. Verfahren nach Anspruch 1 zur Herstellung von exo-2,4-Diamino-5-[8-(3,5-dimethoxyphenyl)-8-azabicyclo[3.2.1]oct-3-yl]pyrimidin.

6. Verfahren nach Anspruch 1 zur Herstellung von exo-2,4-Diamino-5-[8-(4-methoxyphenyl)-8-azabicyclo[3.2.1]-oct-3-yl]pyrimidin, exo-2,4-Diamino-5-[8-(3,4,5-trimethoxyphenyl)-8-azabicyclo[3.2.1]oct-3-yl]pyrimidin, exo-2,4-Diamino-5-[8-(2,5-dimethoxyphenyl)-8-azabicyclo[3.2.1]-oct-3-yl]-pyrimidin, exo-2,4-Diamino-5-[8-(1-naphthyl)-8-azabicyclo[3.2.1]oct-3-yl]-pyrimidin, exo-2,4-Diamino-5-[8-(-2-naphthyl)-8-azabicyclo[3.2.1]oct-3-yl]-pyrimidin.

7. Verfahren nach Anspruch 1 zur Herstellung von 2,4-Diamino-5-[1-(4-methoxyphenyl)-4-piperidinyl]pyrimidin, 2,4-Diamino-5-[1-(3,5-dimethoxyphenyl)-4-piperidinyl]-pyrimidin, 2,4-Diamino-5-[1- (3,4,5-trimethoxyphenyl)-4-piperidinyl]pyrimidin, 2,4-Diamino-5-[1-(p-nitrophenyl)-4-piperidinyl]-pyrimidin, 2,4-Diamino-5-(4-piperidyl)-pyrimidin, 2,4-Diamino-5-[1-(p-chlorphenyl)-4-piperidinyl]-pyrimidin, Äthyl p-[1-(2,4-diamino-5-pyrimidinyl)-4-piperidinyl]benzoat, p-[1-(2,4-Diamino-5-pyrimidinyl)-4-piperidinyl]benzoesäure, 2,4-Diamino-5-[1-(p-cyanophenyl)-4-piperidinyl]-pyrimidin.

8. Verbindungen der allgemeinen Formel

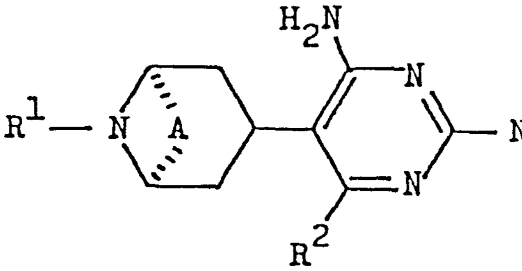

I

worin $R^1$ einen Naphtylrest oder einen Naphtyl- oder Phenylrest darstellt, der durch $C_{1-6}$-Alkyl, $C_{1-6}$Alkylthio, $C_{1-6}$-Alkoxy, Halogen, Amino, $C_{1-6}$-Alkylamino oder di-($C_{1-6}$-Alkyl)-amino, Nitro, Trifluormethyl, $C_{1-6}$-Alkoxycarbonyl, Carboxyl, Cyano oder -CONHR[3], wobei Wasserstoff, $C_{1-6}$-Alkyl oder NHR[3] der über die Aminogruppe gebundene Rest von Glycin, β-Alanin, Asparaginsäure oder Glutaminsäure ist, substituiert ist, $R^2$ Wasserstoff oder $C_{1-6}$-Alkoxy; und A einen fakultativ anwesenden Äthylenrest oder 1,3-Propylenrest darstellen,
und der Diaminopyrimidinrest in Verbindungen der Formel I in denen A anwesend ist, exo-Konfiguration aufweist.

9. Verbindungen gemäss Anspruch 8, in denen $R^1$ ein wie in Anspruch 1 definierter substituierter Phenylrest ist.

10. Verbindungen gemäss Anspruch 8 der allgemeinen Formel

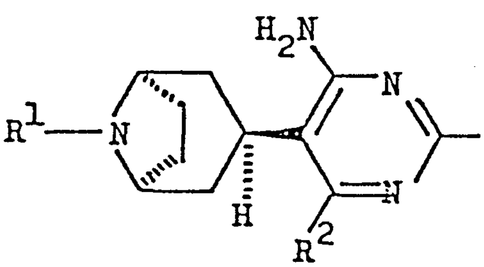

I A

worin $R^1$ die in Anspruch 9 angegebene Bedeutung hat und $R^2$ Wasserstoff oder $C_{1-6}$-Alkoxy ist.

11. Verbindungen gemäss Anspruch 8 der allgemeinen Formel

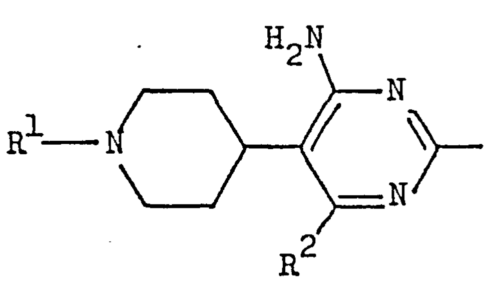

I B

worin $R^1$ die in Anspruch 9 angegebene Bedeutung hat und $R^2$ Wasserstoff oder $C_{1-6}$-Alkoxy ist.

12. exo-2,4-Diamino-5-[8-(3,5-dimethoxyphenyl)-8-azabicyclo[3.2.1]oct-3-yl]pyrimidin.

13. exo-2,4-Diamino-5-[8-(4-methoxyphenyl)-8-azabicyclo]3.2.1]oct-3-yl]pyrimidin, exo-2,4-Diamino-5-[8-(3,4,5-trimethoxyphenyl)-8-azabicyclo[3.2.1]oct-3-yl]pyrimidin, exo-2,4-Diamino-5-[8-(1,5-dimethoxyphenyl)-8-azabicyclo[3.2.1]oct-3-yl]-pyrimidin, exo-2,4-Diamino-5-[8-(1-naphthyl)-8-azabicyclo[3.2.1]oct-3-yl]-pyrimidin, exo-2,4-Diamino-5-[8-(-2-napththyl)-8-azabicyclo[3.2.1]oct-3-yl]-pyrimidin.

14. 2,4-Diamino-5-[1-(4-methoxyphenyl)-4-piperidinyl]-pyrimidin, 2,4-Diamino-5-[1-(3,5-dimethoxyphenyl)-4-piperidinyl]pyrimidin, 2,4-Diamino-5-[1-(3,4,5-trimethoxyphenyl)-4-piperidinyl]pyrimidin, 2,4-Diamino-5-[1-(p-nitrophenyl)-4-piperidinyl]-pyrimidin, 2,4-Diamino-5-(4-piperidyl)-pyrimidin, 2,4-Diamino-5-[1-(p-chlorphenyl)-4-piperidinyl]-pyrimidin, Äthyl p-[1-(2,4-diamino-5-pyrimidinyl)-4-piperidinyl]-benzoat, p-[1-(2,4-Diamino-5-pyrimidinyl)-4-piperidinyl]-benzoesäure, 2,4-Diamino-5-[1-(p-cyanophenyl)-4-piperidinyl]-pyrimidin.

15. Verbindungen der Formel

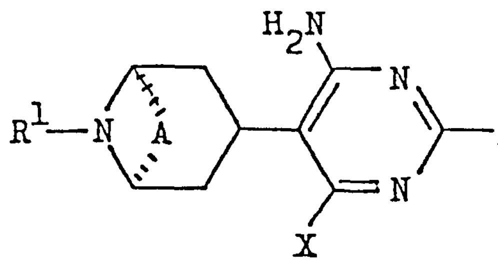

II

worin $R^1$ und A die in Anspruch 8 angegebene Bedeutung haben und X Chlor oder Brom ist.

16. Verbindungen gemäss den Ansprüchen 8–14 zur Anwendung als Heilmittel.

17. Verbindungen der allgemeinen Formel I zur Anwendung bei der Behandlung bakterieller Infektionen oder der Malaria.

18. Pharmazeutisches Präparat, enthaltend eine Verbindung der allgemeinen Formel I und einen Trägerstoff.

**Claims for the Contracting States: BE, CH, DE, FR, GB, IT, LI, LU, NL, SE**

1. Compounds of the general formula

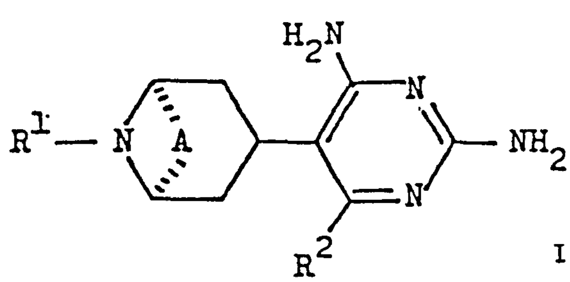

I

wherein $R^1$ represents a napththyl residue or a naphthyl or phenyl residue which is substituted by $C_{1-6}$-alkyl, $C_{1-6}$-alkylthio, $C_{1-6}$-alkoxy, halogen, amino, $C_{1-6}$-alkylamino or di-($C_{1-6}$-alkyl)-amino,

nitro, trifluoromethyl, $C_{1-6}$-alkoxycarbonyl, carboxyl, cyano or -CONHR³ in which R³ is hydrogen, $C_{1-6}$-alkyl or NHR³ is the residue of glycine, β-alanine, aspartic acid or glutamic acid attached via the amino group; R² is hydrogen or $C_{1-6}$-alkoxy; and A represents an optionally present ethylene residue or 1,3-propylene residue, and the diaminopyrimidine residue in compounds of formula I in which A is present has the exo-configuration.

2. Compounds in accordance with claim 1, in which R¹ is a substituted phenyl residue as defined in claim 1.

3. Compounds in accordance with claim 1 of the general formula

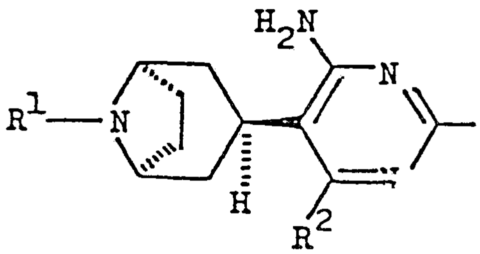

I A

wherein R¹ has the significance given in claim 2 and R² is hydrogen or $C_{1-6}$-alkoxy.

4. Compounds in accordance with claim 1 of the general formula

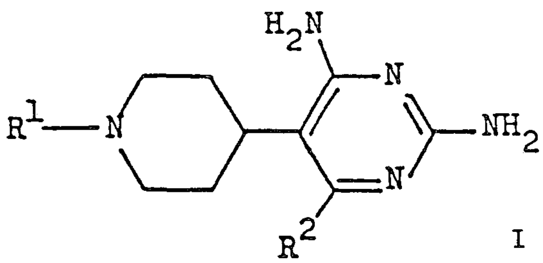

I B

wherein R¹ has the significance given in claim 2 and R² is hydrogen or $C_{1-6}$-alkoxy.

5. exo-2,4-Diamino-5-[8-(3,5-dimethoxyphenyl)-8-azabicyclo[3.2.1]oct-3-yl]pyrimidine.

6. exo-2,4-Diamino-5-[8-(4-methoxyphenyl)-8-azabicyclo-[3.2.1]oct-3-yl]pyrimidine, exo-2,4-diamino-5-[8-(3,4,5-trimethoxyphenyl)-8-azabicyclo[3.2.1]oct-3-yl]pyrimidine, exo-2,4-diamino-5-[8-(2,5-dimethoxyphenyl)-8-azabicyclo[3.2.1]oct-3-yl]-pyrimidine, exo-2,4-diamino-5-[8-(1-naphthyl)-8-azabicyclo[3.2.1]oct-3-yl]-pyrimidine, exo-2,4-diamino-5-[8-(-2-naphthyl)-8-azabicyclo[3.2.1]oct-3-yl]-pyrimidine.

7. 2,4-Diamino-5-[1-(4-methoxyphenyl)-4-piperidinyl]pyrimidine, 2,4-diamino-5-[1-(3,5-dimethoxyphenyl)-4-piperidinyl]pyrimidine, 2,4-diamino-5-[1-(3,4,5-trimethoxyphenyl)-4-piperidinyl]pyrimidine, 2,4-diamino-5-[1-(p-nitrophenyl)-4-piperidinyl]-pyrimidine, 2,4-diamino-5-(4-piperidyl)-pyrimidine, 2,4-diamino-5-[1-(p-chlorophenyl)-4-piperidinyl]-pyrimidine, ethyl p-[1-(2,4-diamino-5-pyrimidinyl)-4-piperidinyl]benzoate, p-[1-(2,4-diamino-5-pyrimidinyl)-4-piperidinyl]benzoic acid, 2,4-diamino-5-[1-(p-cyanophenyl)-4-piperidinyl]pyrimidine.

8. Compounds of the formula

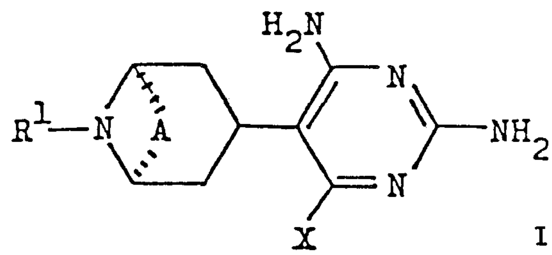

II

wherein R¹ and A have the significance given in claim 1 and X is chlorine or bromine.

9. Compounds in accordance with claims 1-7 for use as medicaments.

10. Compounds of general fomula I for use in the treatment of bacterial infections or of malaria.

11. A process for the manufacture of the compounds of claim 1, characterized by

a) treating a compound of the general formula

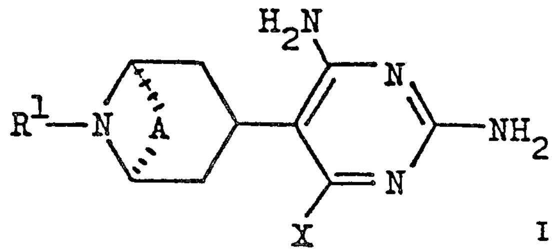

II

wherein X is chlorine or bromine and R¹ and A have the significance given in claim 1, with a reduction agent, or

b) reacting a compound of the general formula

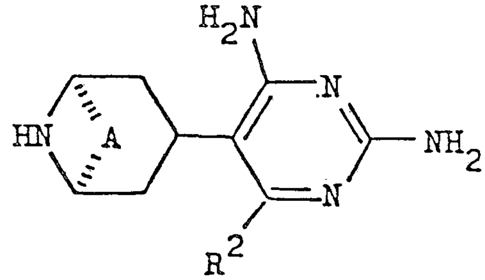

III

wherein R² is hydrogen or $C_{1-6}$-alkoxy, with a compound of the general formula $R^{11}$-Z in which $R^{11}$ is a naphthyl residue or a naphthyl or phenyl residue which is substituted by $C_{1-6}$-alkoxy, halogen, nitro, trifluoromethyl, cyano or $C_{1-6}$-alkoxycarbonyl and Z signifies halogen or nitro, or

c) reacting a compound of general formula II with an alkali metal $C_{1-6}$-alkylate, and, if desired, transforming a substituent present in R¹ in the reaction product into a different substituent present in R¹.

12. A pharmaceutical preparation containing a compound of general formula I and a carrier material.

**Claims for the Contracting State Austria**

1. A process for the manufacture of compounds of the general formula

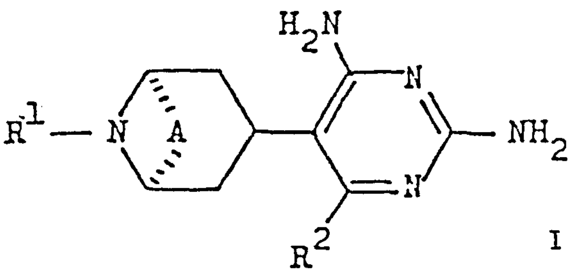

I

wherein $R^1$ represents a naphthyl residue or a naphthyl or phenyl residue which is substituted by $C_{1-6}$-alkyl, $C_{1-6}$-alkylthio, $C_{1-6}$-alkoxy, halogen, amino, $C_{1-6}$-alkylamino or di-($C_{1-6}$-alkyl)-amino, nitro, trifluoromethyl, $C_{1-6}$-alkoxycarbonyl, carboxyl, cyano or -CONHR$^3$ in which $R^3$ is hydrogen, $C_{1-6}$-alkyl or NHR$^3$ is the residue of glycine, β-alanine, aspartic acid or glutamic acid attached via the amino group; $R^2$ is hydrogen or $C_{1-6}$-alkoxy; and A represents an optinally present ethylene residue or 1,3-propylene residue, and the diaminopyrimidine residue in compounds of formula I in which A is present has the exo-configuration, characterized by

a) treating a compound of the general formula

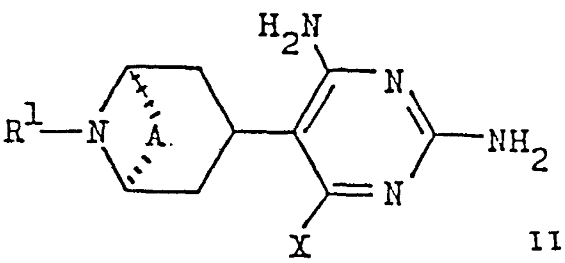

II

wherein X is chlorine or bromine and $R^1$ and A have the significance given in claim 1, with a reduction agent, or

b) reacting a compound of the general formula

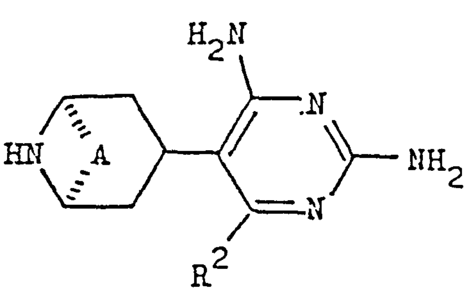

III

wherein $R^2$ is hydrogen or $C_{1-6}$-alkoxy, with a compound of the general formula $R^{11}Z$ in which $R^1$ is a naphthyl residue or a naphthyl or phenyl residue which is substituted by $C_{1-6}$-alkoxy, halogen, nitro, trifluoromethyl, cyano or $C_{1-6}$-alkoxycarbonyl and Z signifies halogen or nitro, or

c) reacting a compound of general formula II with an alkali metal $C_{1-6}$-alkylate,

and, if desired, transforming a substituent present in $R^1$ in the reaction product into a different substituent present in $R^1$.

2. A process according to claim 1 for the manufacture of compounds of general formula I in which $R^1$ is a substituted phenyl residue as defined in claim 1.

3. A process according to claims 1 or 2 for the manufacture of compounds of the general formula

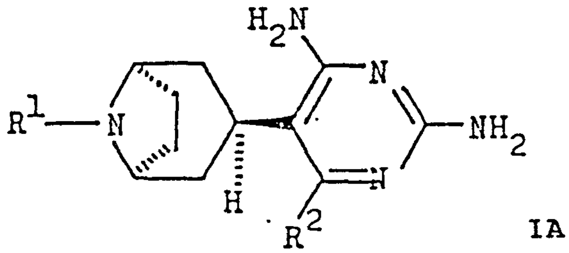

IA

wherein $R^1$ has the significance given in claim 2 and $R^2$ is hydrogen or $C_{1-6}$-alkoxy.

4. A process according to claims 1 or 2 for the manufacture of compounds of the general formula

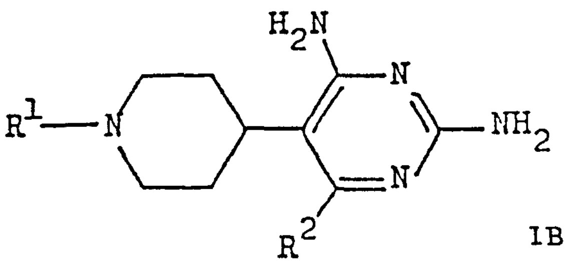

IB

wherein $R^1$ has the significance given in claim 2 and $R^2$ is hydrogen or $C_{1-6}$-alkoxy.

5. A process according to claim 1 for the manufacture of exo-2,4-diamino-5-[8-(3,5-dimethoxyphenyl)-8-azabicyclo-[3.2.1]oct-3-yl]pyrimidine.

6. A process according to claim 1 for the manufacture of exo-2,4-diamino-5-[8-(4-methoxyphenyl)-8-azabicyclo[3.2.1]-oct-3-yl]pyrimidine, exo-2,4-diamino-5-[8-(3,4,5-trimethoxyphenyl)-8-azabicyclo[3.2.1]oct-3-yl]pyrimidine, exo-2,4-diamino-5-[8-,(2,5-dimethoxyphenyl)-8-azabicyclo-[3.2.1]oct-3-yl]-pyrimidine, exo-2,4-diamino-5-[8-(1-naphthyl)-8-azabicyclo[3.2.1]oct-3-yl]-pyrimidine, exo-2,4-diamino-5-[8-(-2-naphthyl)-8-azabicyclo[3.2.1]oct-3-yl]-pyrimidine.

7. A process according to claim 1 for the manufacture of 2,4-diamino-5-[1-(4-methoxyphenyl)-4-piperidinyl]pyrimidine, 2,4-diamino-5-[1-(3,5-dimethoxyphenyl)-4-piperidinyl]pyrimidine, 2,4-diamino-5-[1-(3,4,5-trimethoxyphenyl)-4-piperidinyl]pyrimidine, 2,4-diamino-5-[1-(p-nitrophenyl)-4-piperidinyl]-pyrimidine, 2,4-diamino-5-(4-piperidyl)-pyrimidine, 2,4-diamino-5-[1-(p-chlorophenyl)-4-piperidinyl]-pyrimidine, ethyl p-[1-(2,4-diamino-5-pyrimidinyl)-4-piperidinyl]benzoate, p-[1-(2,4-diamino-5-pyrimidinyl)-4-piperidinyl]benzoic acid, 2,4-diamino-5-[1-(p-cyanophenyl)-4-piperidinyl] pyrimidine.

8. Compounds of the general formula

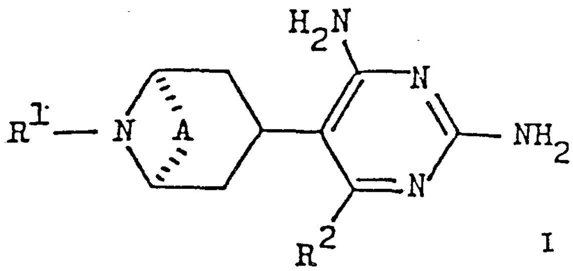

I

wherein $R^1$ represents a naphthyl residue or a naphthyl or phenyl residue which is substituted by $C_{1-6}$-alkyl, $C_{1-6}$-alkylthio, $C_{1-6}$-alkoxy, halogen, amino, $C_{1-6}$-alkylamino or di-($C_{1-6}$-alkyl)-amino, nitro, trifluoromethyl, $C_{1-6}$-alkoxycarbonyl, carboxyl, cyano or -$CONHR^3$ in which $R^3$ is hydrogen, $C_{1-6}$-alkyl or $NHR^3$ is the residue of glycine, β-alanine, aspartic acid or glutamic acid attached via the amino group; $R^2$ is hydrogen or $C_{1-6}$-alkoxy; and A represents an optionally present ethylene residue or 1,3-propylene residue,
and the diaminopyrimidine residue in compounds of formula I in which A is present has the exo-configuration.

9. Compounds in accordance with claim 8, in which $R^1$ is a substituted phenyl residue as defined in claim 1.

10. Compounds in accordance with claim 8 of the general formula

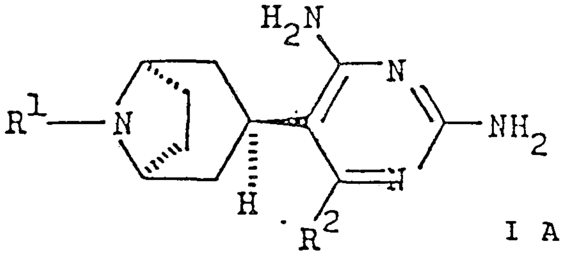

I A

wherein $R^1$ has the significance given in claim 9 and $R^2$ is hydrogen or $C_{1-6}$-alkoxy.

11. Compounds in accordance with claim 8 of the general formula

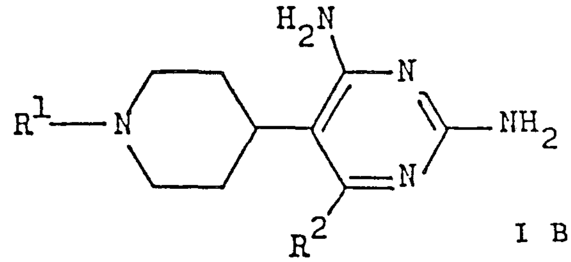

I B

wherein $R^1$ has the significance given in claim 9 and $R^2$ is hydrogen or $C_{1-6}$-alkoxy.

12. exo-2,4-Diamino-5-[8-(3,5-dimethoxyphenyl)-8-azabicyclo[3.2.1]oct-3-yl]pyrimidine.

13. exo-2,4-Diamino-5-[8-(4-methoxyphenyl)-8-azabicyclo-[3.2.1]oct-3-yl]pyrimidine, exo-2,4-diamino-5-[8-(3,4,5-trimethoxyphenyl)-8-azabicyclo[3.2.1]oct-3-yl]pyrimidine, exo-2,4-diamino-5-[8-(2,5-dimethoxyphenyl)-8-azabicyclo-[3.2.1]oct-3-yl]-pyrimidine, exo-2,4-diamino-5-[8-(2-naphthyl)-8-azabicyclo[3.2.1]oct-3-yl]-pyrimidine, exo-2,4-diamino-5-[8-(-2-naphthyl)-8-azabicyclo[3.2.1]oct-3-yl]-pyrimidine.

14. 2,4-Diamino-5-[1-(4-methoxyphenyl)-4-piperidinyl]pyrimidine, 2,4-diamino-5-[1-(3,5-dimethoxyphenyl)-4-piperidinyl]pyrimidine, 2,4-diamino-5-[1-(3,4,5-trimethoxyphenyl)-4-piperidinyl]pyrimidine, 2,4-diamino-5-[1-(p-nitrophenyl)-4-piperidinyl]-pyrimidine, 2,4-diamino-5-(4-piperidyl)-pyrimidine, 2,4-diamino-5-[1-(p-chlorophenyl)-4-piperidinyl]-pyrimidine, ethyl p-[1-(2,4-diamino-5-pyrimidinyl)-4-piperidinyl]benzoate, p-[1-(2,4-diamino-5-pyrimidinyl)-4-piperidinyl]benzoic acid, 2,4-diamino-5-[1-(p-cyanophenyl)-4-piperidinyl]pyrimidine.

15. Compounds of the formula

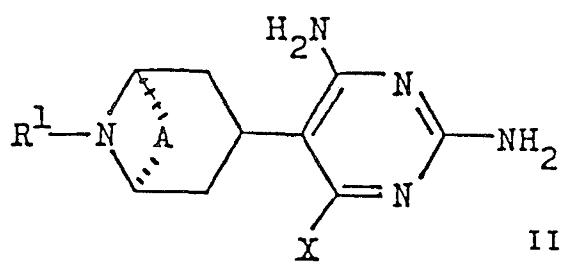

II

wherein $R^1$ and A have the significance given in claim 8 and X is chlorine or bromine.

16. Compounds in accordance with claims 8–14 for use as medicaments.

17. Compounds of general formula I for use in the treatment of bacterial infections or of malaria.

18. A pharmaceutical preparation containing a compound of general formula I and a carrier material.

**Revendications pour les Etats contractants: BE, CH, DE, FR, GB, IT, LI, LU, NL, SE**

1. Composés de formule générale

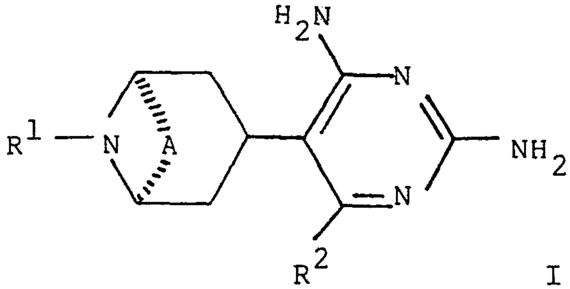

I

dans laquelle R[1] représente un groupe naphtyle ou un groupe naphtyle ou phényle substitué par des groupes alkyle en C 1-C 6, alkylthio en C1–C 6, alcoxy en C 1-C 6, des halogènes, des groupes amino, alkylamino en C 1-C 6 ou di-(alkyle en C 1–C 6)-amino, nitro, trifluorométhyle, (alcoxy en C 1–C 6)-carbonyle, carboxyle, cyano ou -CONHR[3] dans lequel R[3] représente l'hydrogène, un groupe alkyle en C 1-C 6 ou bien NHR[3] représente le reste de la glycine, de la bêta-alanine, de l'acide aspartique ou de l'acide glutamique relié par l'intermédiaire du groupe amino,
R[2] représente l'hydrogène ou un groupe alcoxy en C 1–C 6 et A un groupe éthylène ou 1,3-propylène dont la présence est facultative,
et dans les composés de formule I dans lesquels A est présent, le reste de diaminopyrimidine est en configuration exo.

2. Composés selon la rev. 1, dans lesquels R[1] représente un groupe phényle substitué comme indiqué dans la rev. 1.

3. Composés selon la rev. 1, répondant à la formule générale

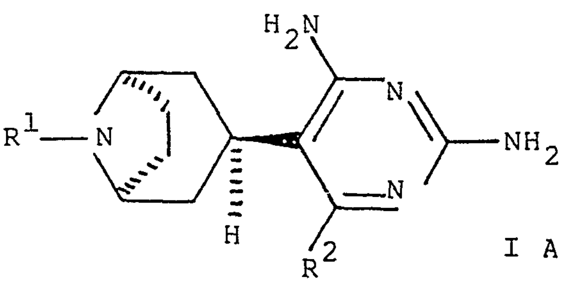

I A

dans laquelle R[1] a les significations indiquées dans la rev. 2 et R[2] représente l'hydrogène ou un groupe alcoxy en C 1-C 6.

4. Composés selon la rev. 1, répondant à la formule générale

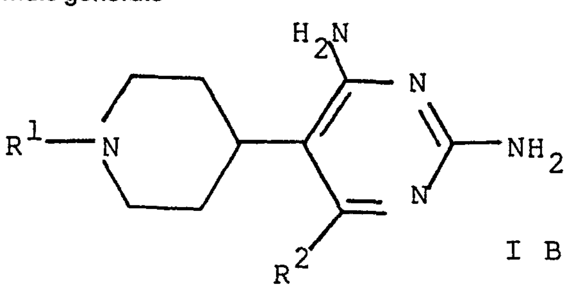

I B

dans laquelle R[1] a les significations indiquées dans la rev. 2 et R[2] représente l'hydrogène ou un groupe alcoxy en C 1-C 6.

5. L'exo-2,4-diamino-5-[8-(3,5-diméthoxyphényl)-8-azabicyclo[3.2.1]octa-3-yl]pyrimidine.

6. L'exo-2,4-diamino-5-[8-(4-méthoxyphényl)-8-azabicyclo[3.2.1]octa-3-yl]pyrimidine, l'exo-2,4-diamino-5-[8-(3,4,5-triméthoxyphényl)-8-azabicyclo[3.2.1]-octa-3-yl]-pyrimidine, l'exo-2,4-diamino-5-[8-(2,5-diméthoxyphényl)-8-azabicyclo[3.2.1]octa-3-yl]-pyrimidine, l'exo-2,4-diamino-5-[8-(1-naphtyl)-8-azabicyclo[3.2.1]-octa-3-yl]-pyrimidine, l'exo-2,4-diamino-5-[8-(2-naphtyl)-8-azabicyclo[3.2.1]octa-3-yl]-pyrimidine.

7. La 2,4-diamino-5-[1-(4-méthoxyphényl)-4-pipéridinyl]-pyrimidine, la 2,4-diamino-5-[1-(3,5-diméthoxyphényl)-4-pipéridinyl]-pyrimidine, la 2,4-diamino-5-[1-(3,4,5-triméthoxyphényl)-4-pipéridinyl]-pyrimidine, la 2,4-diamino-5-[1-(p-nitrophényl)-4-pipéridinyl]-pyrimidine, la 2,4-diamino-5-(pipéridyl)-pyrimidine, la 2,4-diamino-5-[1-(p-chlorophényl)-4-pipéridinyl]-pyrimidine, le p-[1-(2,4-diamino-5-pyrimidinyl)-4-pipéridinyl]-benzoate d'éthyle, l'acide p-[1-(2,4-diamino-5-pyrimidinyl)-4-pipéridinyl]-benzoïque, la 2,4-diamino-5-[1-(p-cyanophényl)-4-pipéridinyl]-pyrimidine.

8. Composés de formule

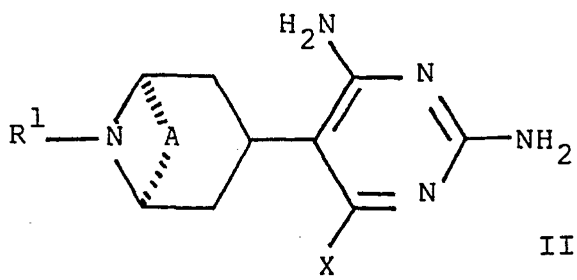

II

dans laquelle R[1] et A ont les significations indiquées dans la rev. 1, et X représente le chlore ou le brome.

9. Composés selon les rev. 1 à 7, pour l'utilisation en tant que médicaments.

10. Composés de formule générale I pour l'utilisation dans le traitement des infections bactériennes ou de la malaria.

11. Procédé de préparation des composés de la rev. 1, caractérisé en ce que:

a) on traite par un agent réducteur un composé de formule générale

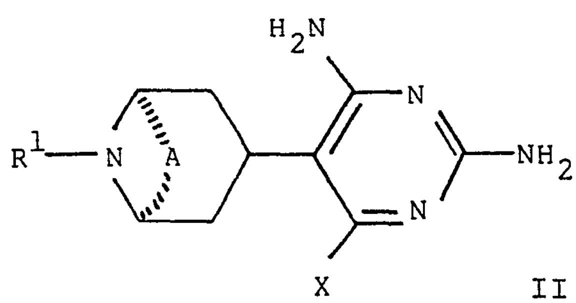

II

dans laquelle X représente le chlore ou le brome et R[1] et A ont les significations indiquées dans la rev. 1, ou bien

b) on fait réagir un composé de formule générale

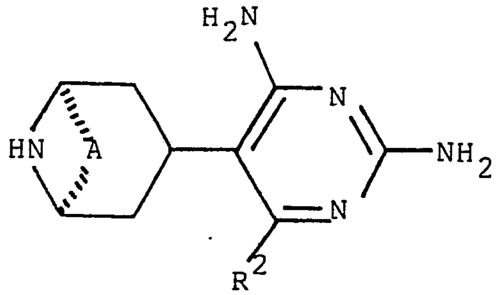

III

dans laquelle $R^2$ représente l'hydrogène ou un groupe alcoxy en C 1-C 6, avec un composé de formule générale $R^{11}$-Z dans laquelle $R^{11}$ représente un groupe naphtyle ou un groupe naphtyle ou phényle substitué par des groupes alcoxy en C 1-C 6, des halogènes, des groupes nitro, trifluorométhyle, cyano ou (alcoxy en C 1-C 6)-carbonyle et Z représente un halogène ou un groupe nitro, ou bien

c) on fait réagir un composé de formule générale II avec un alkylate en C 1-C 6 de métal alcalin, et, si on le désire, dans le produit de réaction, on convertit un substituant contenu dans $R^1$ en un autre substituant contenu dans $R^1$.

12. Composition pharmaceutique contenant un composé de formule générale I et un véhicle.

**Revendications pour l'Etat contractant: AT**

1. Procédé de préparation des composés de formule générale

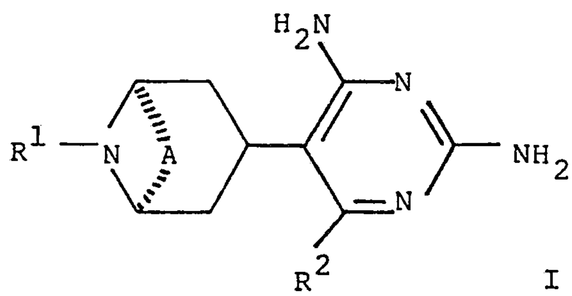

I

dans laquelle $R^1$ représente un groupe naphtyle ou un groupe naphthyle ou phényle substitué par des groupes alkyle en C 1-C 6, alkylthio en C 1-C 6, alcoxy en C 1-C 6, des halogènes, des groupes amino, alkylamino en C 1-C 6 ou di-(alkyle en C 1-C 6)-amino, nitro, trifluorométhyle, (alcoxy en C 1-C 6)-carbonyle, carboxyle, cyano ou -$CONHR^3$ dans lequel $R^3$ représente l'hydrogène, un groupe alkyle en C 1-C 6 ou bien $NHR^3$ représente le reste de la glycine, de la bêta-alanine, de l'acide aspartique ou de l'acide glutamique relié par l'intermédiaire du groupe amino,

$R^2$ représente l'hydrogène ou un groupe alcoxy en C 1-C 6 et A un groupe éthylène ou 1,3-propylène dont la présence est facultative,

et dans les composés de formule I dans lesquels A est présent, le reste de diaminopyrimidine est en configuration exo, caractérisé en ce que:

a) on traite par un agent réducteur un composé de formule générale

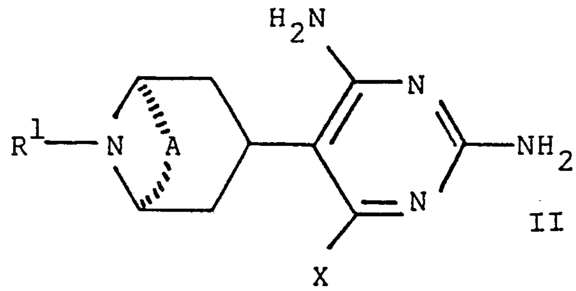

II

dans laquelle X représente le chlore ou le brome et $R^1$ et A ont les significations indiquées dans la rev. 1, ou bien

b) on fait réagir un composé de formule générale

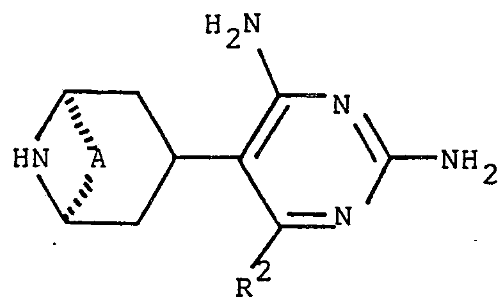

III

dans laquelle $R^2$ représente l'hydrogène ou un groupe alcoxy en C 1-C 6, avec un composé de formule générale $R^{11}$-Z dans laquelle $R^{11}$ représente un groupe naphtyle ou un groupe naphtyle ou phényle substitué par des groupes alcoxy en C 1-C 6, des halogènes, des groupes nitro, trifluorométhyle, cyano ou (alcoxy en C 1-C 6)-carbonyle et Z représente un halogène ou un groupe nitro, ou bien

c) on fait réagir un composé de formule générale II avec un alkylate en C 1-C 6 de métal alcalin, et, si on le désire, dans le produit de réaction, on convertit un substituant contenu dans $R^1$ en un autre substituant contenu dans $R^1$.

2. Procédé selon la revendication 1 pour la préparation des composés de formule générale I dans laquelle $R^1$ représente un groupe phényle substitué comme indiqué dans la revendication 1.

3. Procédé selon la revendication 1 ou 2 pour la préparation des composés répondant à la formule générale

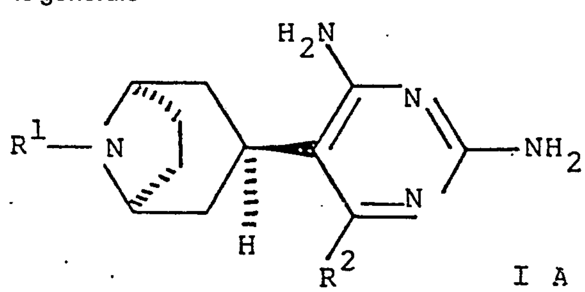

I A

dans laquelle $R^1$ a les significations indiquées dans la rev. 2 et $R^2$ représente l'hydrogène ou un groupe alcoxy en C 1-C 6.

4. Procédé selon la revendication 1 ou 2 pour la préparation des composés répondant à la formule générale

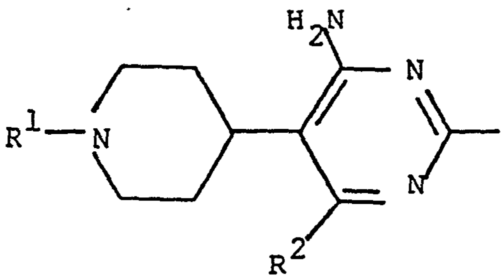

I B

dans laquelle $R^1$ a les significances indiquées dans la revendication 2 et $R^2$ représente l'hydrogène ou un groupe alcoxy en C 1-C 6.

5. Procédé selon la revendication 1 pour la préparation de l'exo-2,4-diamino-5-[8-(3,5-diméthoxyphényl)-8-azabicyclo[3.2.1]octa-3-yl]pyrimidine.

6. Procédé selon la revendication 1 pour la préparation de l'exo-2,4-diamino-5-[8-(4-méthoxyphényl)-8-azabicyclo[3.2.1]octa-3-yl]pyrimidine, l'exo-2,4-diamino-5-[8-(3,4,5-triméthoxyphényl)-8-azabicyclo[3.2.1]-octa-3-yl]-pyrimidine, l'exo-2,4-diamino-5-[8-(2,5-diméthoxyphényl)-8-azabicyclo[3.2.1]octa-3-yl]-pyrimidine, l'exo-2,4-diamino-5-[8-(1-naphtyl)-8-azabicyclo[3.2.1]-octa-3-yl]-pyrimidine, l'exo-2,4-diamino-5-[8-(2-naphtyl)-8-azabicyclo[3.2.1]octa-3-yl]-pyrimidine.

7. Procédé selon la revendication 1 pour la préparation de la 2,4-diamino-5-[1-(4-méthoxyphényl)-4-pipéridinyl]-pyrimidine, la 2,4-diamino-5-[1-(3,5-diméthoxyphényl)-4-pipéridinyl]-pyrimidine, la 2,4-diamino-5-[1-(3,4,5-triméthoxyphényl)-4-pipéridinyl]-pyrimidine, la 2,4-diamino-5-[1-(p-nitrophényl)-4-pipéridinyl]-pyrimidine, la 2,4-diamino-5-(-pipéridyl)-pyrimidine, la 2,4-diamino-5-[1-(p-chlorophényl)-4-pipéridinyl)-pyrimidine, le p-[1-(2,4-diamino-5-pyrimidinyl)-4-pipéridinyl]-benzoate d'éthyle, l'acide p-[1-(2,4-diamino-5-pyrimidinyl)-4-pipéridinyl]-benzoïque, la 2,4-diamino-5-[1-(p-cyanophényl)-4-pipéridinyl]-pyrimidine.

8. Composés de formule générale

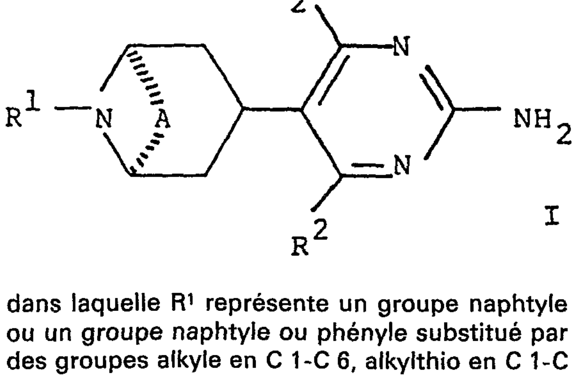

I

dans laquelle $R^1$ représente un groupe naphtyle ou un groupe naphtyle ou phényle substitué par des groupes alkyle en C 1-C 6, alkylthio en C 1-C 6, alcoxy en C 1-C 6, des halogènes, des groupes amino, alkylamino en C 1-C 6 ou di-(alkyle en C 1-C 6)-amino, nitro, trifluorométhyle, (alcoxy en C 1-C 6)-carbonyle, carboxyle, cyano ou -$CONHR^3$ dans lequel $R^3$ représente l'hydrogène, un groupe alkyle en C 1-C 6 ou bien $NHR^3$ représente le reste de la glycine, de la bêta-alanine, de l'acide aspartique ou de l'acide glutamique relié par l'intermédiaire du groupe amino, $R^2$ représente l'hydrogène ou un groupe alcoxy en C 1-C 6 et A un groupe éthylène ou 1,3-propylène dont la présence est facultative, et dans les composés de formule I dans lesquels A est présent, le reste de diaminopyrimidine est en configuration exo.

9. Composés selon la rev. 8, dans lesquels $R^1$ représente un groupe phényle substitué comme indiqué dans la rev. 1.

10. Composés selon la rev. 8, répondant à la formule générale

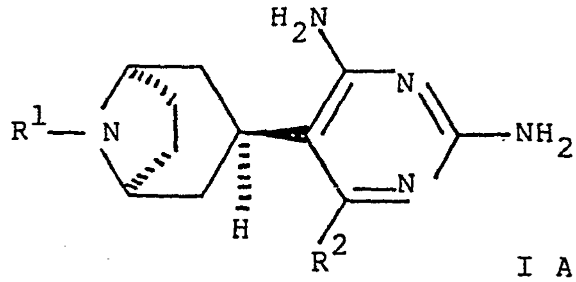

I A

dans laquelle $R^1$ a les significations indiquées dans la rev. 9 et $R^2$ représente l'hydrogène ou un groupe alcoxy en C 1-C 6.

11. Composés selon la rev. 8, répondant à la formule générale

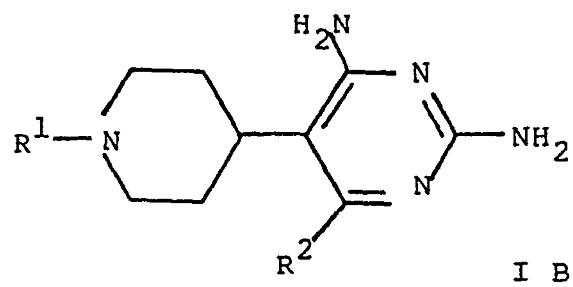

I B

dans laquelle $R^1$ a les significations indiquées dans la rev. 9 et $R^2$ représente l'hydrogène ou un groupe alcoxy en C 1-C 6.

12. L'exo-2,4-diamino-5-[8-(3,5-diméthoxyphényl)-8-azabicyclo[3.2.1]octa-3-yl]pyrimidine.

13. L'exo-2,4-diamino-5-[8-(4-méthoxyphényl)-8-azabicyclo[3.2.1]octa-3-yl]pyrimidine, l'exo-2,4-diamino-5-[8-(3,4,5-triméthoxyphényl)-8-azabicyclo[3.2.1]-octa-3-yl]-pyrimidine, l'exo-2,4-diamino-5-[8-(2,5-diméthoxyphé-

nyl)-8-azabicyclo[3.2.1]octa-3-yl]-pyrimidine, l'exo-2,4-diamino-5-[8-(1-naphtyl)-8-azabicyclo[3.2.1]-octa-3-yl]-pyrimidine, l'exo-2,4-diamino-5-[8-(2-naphtyl)-8-azabicyclo[3.2.1]octa-3-yl]-pyrimidine.

14. La 2,4-diamino-5-[1-(4-méthoxyphényl)-4-pipéridinyl]-pyrimidine, la 2,4-diamino-5-[1-(3,5-diméthoxyphényl)-4-pipéridinyl]-pyrimidine, la 2,4-diamino-5-[1-(3,4,5-triméthoxyphényl)-4-pipéridinyl]-pyrimidine, la 2,4-diamino-5-[1-(p-nitrophényl)-4-pipéridinyl]-pyrimidine, la 2,4-diamino-5-(pipéridyl)-pyrimidine, la 2,4-diamino-5-[1-(p-chlorophényl)-4-pipéridinyl]-pyrimidine, le p-[1-(2,4-diamino-5-pyrimidnyl)-4-pipéridinyl]-benzoate d'éthyle, l'acide p-[1-(2,4-diamino-5-pyrimidinyl)-4-pipéridinyl]-benzoïque, la 2,4-diamino-5-[1-(p-cyanophényl)-4-pipéridinyl]-pyrimidine.

15. Composés de formule

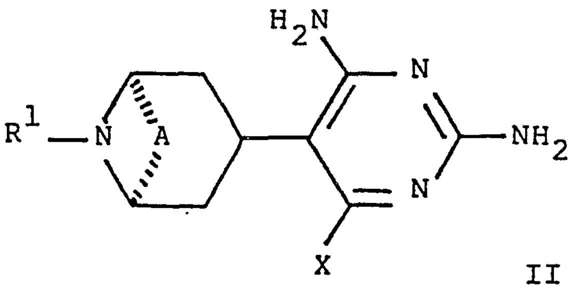

II

dans laquelle R[1] et A ont les significations indiquées dans la rev. 8, et X représente le chlore ou le brome.

16. Composés selon les rev. 8 à 14, pour l'utilisation en tant que médicaments.

17. Composés de formule générale I pour l'utilisation dans le traitement des infections bactériennes ou de la malaria.

18. Composition pharmaceutique contenant un composé formule générale I et un véhicule.